(19) Europäisches Patentamt
European Patent Office
Office européen des brevets

(11) Veröffentlichungsnummer: **0 310 550 B1**

(12) **EUROPÄISCHE PATENTSCHRIFT**

(45) Veröffentlichungstag der Patentschrift: **26.05.93**

(51) Int. Cl.[5]: **C07D 239/42**, C07D 239/30, C07D 239/38, A01N 43/54

(21) Anmeldenummer: **88810640.8**

(22) Anmeldetag: **20.09.88**

(54) **Schädlingsbekämpfungsmittel.**

(30) Priorität: **28.09.87 CH 3750/87**
**11.04.88 CH 1333/88**

(43) Veröffentlichungstag der Anmeldung:
**05.04.89 Patentblatt 89/14**

(45) Bekanntmachung des Hinweises auf die Patenterteilung:
**26.05.93 Patentblatt 93/21**

(84) Benannte Vertragsstaaten:
**AT BE CH DE ES FR GB GR IT LI LU NL SE**

(56) Entgegenhaltungen:
**EP-A- 0 013 143**
**EP-A- 0 135 472**
**EP-A- 0 172 786**
**US-A- 3 499 898**

**PATENT ABSTRACTS OF JAPAN, Band 5, Nr. 132(C-68)[804], 1981**

(73) Patentinhaber: **CIBA-GEIGY AG**
**Klybeckstrasse 141**
**CH-4002 Basel(CH)**

(72) Erfinder: **Hubele, Adolf, Dr.**
**Obere Egg 9**
**CH-4312 Magden(CH)**

EP 0 310 550 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 2-Anilino-pyrimidin-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Insekten und pflanzenschädigenden Mikroorganismen, vorzugsweise Pilzen.

Die erfindungsgemässen Verbindungen entsprechen der allgemeinen Formel I

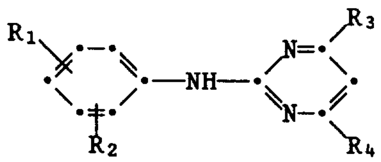

(I)

in welcher bedeuten:

$R_1$ Wasserstoff, Halogen, $C_1-C_3$-Alkyl, Trifluormethyl, $C_1-C_3$-Alkoxy oder $C_1-C_3$-Halogenalkoxy;

$R_2$ Wasserstoff, Halogen, $C_1-C_3$-Alkyl, Trifluormethyl oder $C_1-C_3$-Alkoxy;

$R_3$ Wasserstoff, $C_1-C_4$-Alkyl; oder durch Halogen oder Hydroxy substituiertes $C_1-C_4$-Alkyl; Cyclopropyl oder durch Methyl oder Halogen bis zu dreifach gleich oder verschieden substituiertes Cyclopropyl;

$R_4$ $C_3-C_6$-Cycloalkyl oder durch Methyl und/oder Halogen bis zu dreifach gleich oder verschieden substituiertes $C_3-C_6$-Cycloalkyl; unter Einschluss ihrer Säureadditionssalze und Metallsalzkomplexe.

Unter Alkyl selbst oder als Bestandteil eines anderen Substituenten, wie Alkoxy oder Halogenalkoxy, sind je nach Arizahl der benannten Kohlenstoffatome beispielsweise zu verstehen Methyl, Ethyl, Propyl, Butyl, sowie ihre Isomeren, wie z.B. Isopropyl, Isobutyl, tert.-Butyl oder sek.-Butyl. Halogen, auch Hal genannt, steht für Fluor, Chlor, Brom oder Jod. Halogenalkoxy bezeichnet über Sauerstoff gebundene einfach bis perhalogenierte Reste, wie z.B. $CHCl_2$, $CH_2F$, $CCl_3$, $CH_2Cl$, $CHF_2$, $CF_3$, $CH_2CH_2Br$, $C_2Cl_5$, $CH_2Br$, CHBrCl usw., vorzugsweise $CF_3$. Cycloalkyl steht je nach Zahl der genannten Kohlenstoffatome z.B. für Cyclopropyl, Cyclobutyl, Cyclopentyl oder Cyclohexyl.

N-Pyrimidinylanilinverbindungen sind bereits bekannt. So sind in der publizierten europäischen Patentanmeldung 0 224 339 und der DD-Patentschrift 151 404 Verbindungen, die eine N-2-Pyrimidinylstruktur aufweisen, als wirksam gegen pflanzenschädigende Fungi beschrieben. Die bekannten Verbindungen konnten jedoch bisher die in der Praxis an sie gestellten Forderungen nicht in vollem Masse befriedigen. Ferner sind Nitroanilinpyrimidin-Verbindungen mit fungizider Wirksamkeit in den europäischen Patentanmeldungen EP-A-0 172 786 und EP-A-0 135 472 und solche mit insektizider Wirksamkeit in der japanischen Patentanmeldung 54-141 647 [Vgl. Patent Abstract of Japan, Band 5, Nr. 132 (C. 68) (804) 1981] beschrieben. Weitere Anilinoderivate mit herbizider Wirksamkeit sind in der europäischen Patentanmeldung EP-A-0 013 143 offenbart. Die erfindungsgemässen Verbindungen der Formel I unterscheiden sich in charakteristischer Weise von den bekannten Verbindungen durch die Einführung mindestens eines Cycloalkyl-Restes und anderer Substituenten in die Anilinopyrimidin-Struktur, wodurch bei den neuen Verbindungen eine unerwartet hohe fungizide Wirksamkeit und insektizide Wirkung erreicht wird.

Die Verbindungen der Formel I sind bei Raumtemperatur stabile Oele, Harze oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende insektizide und fungizide Wirkung, sondern auch durch besonders gute Pflanzenverträglichkeit aus.

Die Erfindung betrifft sowohl die freien Verbindungen der Formel I als auch deren Additionssalze mit anorganischen und organischen Säuren sowie deren Komplexe mit Metallsalzen.

Erfindungsgemässe Salze sind insbesondere Additionssalze mit unbedenklichen anorganischen oder organischen Säuren, beispielsweise Halogenwasserstoffsäuren, z.B. Chlor-, Brom- oder Jodwasserstoffsäure, Schwefelsäure, Phosphorsäure, phosphorige Säure, Salpetersäure, oder organischen Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicyclsäure, 2-Phenoxybenzoesäure, 2-

Acetoxybenzoesäure oder 1,2 – Naphthalin – disulfonsäure.

Metallsalzkomplexe der Formel I bestehen aus dem zugrundeliegenden organischen Molekül und einem anorganischen oder organischen Metallsalz, z.B. den Halogeniden, Nitraten, Sulfaten, Phosphaten, Acetaten, Trifluoracetaten, Trichloracetaten, Propionaten, Tartraten, Sulfonaten, Salicyclaten, Benzoaten usw. der Elemente der zweiten Hauptgruppe wie Calcium und Magnesium und der dritten und vierten Hauptgruppe wie Aluminium, Zinn oder Blei sowie der ersten bis achten Nebengruppe wie Chrom, Mangan, Eisen, Kobalt, Nickel, Kupfer, Zink usw. Bevorzugt sind die Nebengruppen – Elemente der 4. Periode. Die Metalle können dabei in den verschiedenen ihnen zukommenden Wertigkeiten vorliegen. Die Metallkomplexe können ein – oder mehrkernig auftreten, d.h. sie können ein oder mehrere organische Molekülanteile als Liganden enthalten.

Eine wichtige Gruppe von Pflanzenfungiziden und Insektiziden sind jene der Formel I, bei denen $R_1$ und $R_2$ Wasserstoff bedeuten.

Eine besondere Gruppe stellen folgende Verbindungen der Formel I dar, in denen $R_1$ Wasserstoff, Halogen, $C_1 - C_3$ – Alkyl, Trifluormethyl, $C_1 - C_3$ – Alkoxy oder $C_1 - C_3$ – Halogenalkoxy und $R_2$ Wasserstoff, Halogen, $C_1 - C_3$ – Alkyl, Trifluormethyl oder $C_1 - C_3$ – Alkoxy;
$R_3$ Wasserstoff, $C_1 - C_4$ – Alkyl oder durch Halogen substituiertes $C_1 - C_4$ – Alkyl; und
$R_4$ $C_3 - C_6$ – Cycloalkyl oder durch Methyl oder Halogen substituiertes $C_3 - C_6$ – Cycloalkyl bedeuten.

Folgende Wirkstoffgruppen sind aufgrund ihrer ausgeprägten mikrobiziden, insbesondere pflanzenfungiziden, Aktivität bevorzugt:

<u>Gruppe 1a</u>: Verbindungen der Formel, worin bedeuten:

$R_1$ Wasserstoff, Fluor, Chlor, Brom, Methyl, Ethyl, Trifluormethyl, Methoxy, Ethoxy oder Halogenmethoxy;
$R_2$ Wasserstoff, Flour, Chlor, Methyl, Trifluromethyl, Methoxy oder Ethoxy;
$R_3$ Wasserstoff, Methyl, durch Fluor, Chlor, Brom oder Cyano substituiertes Methyl; Ethyl, durch Fluor, Chlor, Brom oder Cyano substituiertes Ethyl; n – Propyl oder sek. – Butyl;
$R_4$ $C_3 - C_6$ – Cycloalkyl oder durch Methyl, Fluor, Chlor oder Brom substituiertes $C_3 - C_6$ – Cycloalkyl.

Unter den vorstehend genannten Verbindungen stellen jene eine besonders bevorzugte Gruppe dar, bei denen $R_1 = R_2 =$ Wasserstoff ist (= Gruppe 1aa).

<u>Gruppe 1b</u>: Verbindungen der Formel I, worin bedeuten:

$R_1$ und $R_2$ unabhängig voneinander Wasserstoff, Chlor, Brom, Methyl, Ethyl, Trifluormethyl, Methoxy, Ethoxy oder Difluormethoxy;
$R_3$ Wasserstoff, Methyl, durch Fluor oder Chlor substituiertes Methyl, Ethyl oder n – Propyl;
$R_4$ $C_3 - C_5$ – Cycloalkyl oder durch Methyl oder Chlor substituiertes $C_3 - C_5$ – Cycloalkyl.

Unter den vorstehend genannten Verbindungen stellen jene eine besonders bevorzugte Gruppe dar, bei denen $R_1 = R_2 =$ Wasserstoff ist (= Gruppe 1bb).

<u>Gruppe 1c</u>: Verbindungen der Formel I, worin bedeuten:

$R_1$ und $R_2$ unabhängig voneinander Wasserstoff, Chlor, Methyl, Methoxy, Ethoxy oder Trifluormethyl;
$R_3$ Wasserstoff, Methyl, Ethyl oder Trifluormethyl;
$R_4$ Cyclopropyl oder durch Methyl oder Chlor substituiertes Cyclopropyl.

Unter den vorstehend genannten Verbindungen stellen jene eine besonders bevorzugte Gruppe dar, bei denen $R_1 = R_2 =$ Wasserstoff ist (= Gruppe 1cc).

<u>Gruppe 1d</u>: Verbindungen der Formel I, worin bedeuten:

$R_1$ Wasserstoff;
$R_2$ und $R_3$ unabhängig voneinander Wasserstoff oder Methyl;
$R_4$ Cyclopropyl oder durch Methyl substituiertes Cyclopropyl.

Gruppe 2aa: Verbindungen der Formel I, worin bedeuten:

$R_1$ und $R_2$ Wasserstoff;
$R_3$ Wasserstoff, $C_1-C_4$-Alkyl; durch Halogen oder Hydroxy substituiertes $C_1-C_2$-Alkyl; Cyclopropyl oder durch Methyl und/oder Halogen bis zu dreifach gleich oder verschieden substituiertes Cyclopropyl;
$R_4$ $C_3-C_6$-Cycloalkyl oder durch Methyl und/oder Halogen bis zu dreifach gleich oder verschieden substituiertes $C_3-C_4$-Cycloalkyl.

Gruppe 2bb: Verbindungen der Formel I, worin bedeuten:

$R_1$ und $R_2$ Wasserstoff;
$R_3$ Wasserstoff, $C_1-C_3$-Alkyl, durch Halogen oder Hydroxy substituiertes $C_1-C_2$-Alkyl; Cyclopropyl oder durch Methyl und/oder Halogen bis zu dreifach gleich oder verschieden substituiertes Cyclopropyl;
$R_4$ $C_3-C_6$-Cycloalkyl oder durch Methyl und/oder Halogen bis zu dreifach gleich oder verschieden substituiertes $C_3-C_4$-Cycloalkyl.

Gruppe 2c: Verbindungen der Formel I, worin bedeuten:

$R_1$ Wasserstoff, Fluor, Chlor, Methyl, Trifluormethyl, Methoxy oder Difluormethoxy;
$R_2$ Wasserstoff, Fluor, Chlor, Methyl, Trifluormethyl oder Methoxy;
$R_3$ Wasserstoff, $C_1-C_3$-Alkyl; durch Halogen oder Hydroxy substituiertes $C_1-C_2$-Alkyl; Cyclopropyl oder durch Methyl und/oder Halogen bis zu dreifach gleich oder verschieden substituiertes Cyclopropyl;
$R_4$ $C_3-C_6$-Cycloalkyl oder durch Methyl und/oder Halogen bis zu dreifach gleich oder verschieden substituiertes $C_3-C_4$-Cycloalkyl.

Unter den vorstehend genannten Verbindungen stellen jene eine besonders bevorzugte Gruppe dar, bei denen $R_1 = R_2 =$ Wasserstoff ist (= Gruppe 2cc).

Gruppe 2d: Verbindungen der Formel I, worin bedeuten:

$R_1$ und $R_2$ Wasserstoff;
$R_3$ $C_1-C_3$-Alkyl, durch Fluor, Chlor, Brom oder Hydroxy substituiertes Methyl; Cyclopropyl, durch Methyl, Fluor, Chlor oder Brom substituiertes Cyclopropyl;
$R_4$ $C_3-C_4$-Cycloalkyl oder durch Methyl und/oder Fluor, Chlor, Brom bis zu dreifach gleich oder verschieden substituiertes $C_3-C_4$-Cycloalkyl.

Unter den besonders bevorzugten Einzelsubstanzen sind z.B. zu nennen:
2-Phenylamino-4-methyl-6-cyclopropyl-pyrimidin (Verb. Nr. 1.1)
2-Phenylamino-4-ethyl-6-cyclopropyl-pyrimidin (Verb. Nr. 1.6)
2-Phenylamino-4-methyl-6-(2-methylcyclopropyl)-pyrimidin (Verb. Nr. 1.14)
2-Phenylamino-4,6-bis(cyclopropyl)pyrimidin (Verb. Nr. 1.236)
2-Phenylamino-4-hydroxymethyl-6-cyclopropyl-pyrimidin (Verb. Nr. 1.48)
2-Phenylamino-4-fluormethyl-6-cyclopropyl-pyrimidin (Ver. Nr. 1.59)
2-Phenylamino-4-hydroxymethyl-6-(2-methylcyclopropyl)-pyrimidin (Verb. Nr. 1.13)
2-Phenylamino-4-methyl-6-(2-fluorcyclopropyl)-pyrimidin (Verb. Nr. 1.66)
2-Phenylamino-4-methyl-6-(2-chlorcyclopropyl)-pyrimidin (Verb. Nr. 1.69)
2-Phenylamino-4-methyl-6-(2-difluorcyclopropyl)-pyrimidin (Verb. Nr. 1.84)
2-Phenylamino-4-fluormethyl-6-(2-fluorcyclopropyl)-pyrimidin (Verb. Nr. 1.87)
2-Phenylamino-4-fluormethyl-6-(2-chlorcyclopropyl)-pyrimidin (Verb. Nr. 1.94)
2-Phenylamino-4-fluormethyl-6-(2-methylcyclopropyl)-pyrimidin (Verb. Nr. 1.108)
2-Phenylamino-4-ethyl-6-(2-methylcyclopropyl)-pyrimidin (Verb. Nr. 1.131)
2-(p-Fluorphenylamino)-4-methyl-6-cyclopropyl-pyrimidin (Verb. Nr. 1.33).

Die Verbindungen der Formel I lassen sich hergestellen, indem man

1. ein Phenylguanidinsalz der Formel IIa

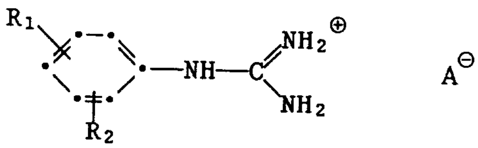

(IIa)

oder das zugrundeliegende Guanidin der Formel IIb

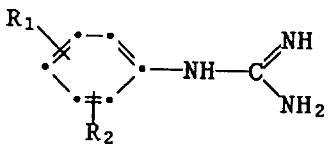

(IIb)

mit einem Diketon der Formel III

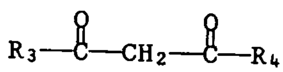

(III)

ohne Lösungsmittel oder in einem aprotischen, bevorzugt in einem protischen Lösungsmittel bei Temperaturen von 60 ° C bis 160 ° C, bevorzugt 60 ° C bis 110 ° C, umsetzt oder

2. in einem mehrstufigen Verfahren:

2.1 Harnstoff der Formel IV

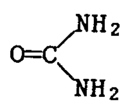

(IV)

mit einem Diketon der Formel III

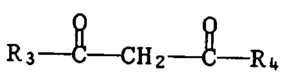

(III)

in Gegenwart einer Säure in einem inerten Lösungsmittel bei Temperaturen von 20 ° C bis 140 ° C, bevorzugt 20 ° C bis 40 ° C, zur Reaktion bringt und zu einer Pyrimidinverbindung der Formel V

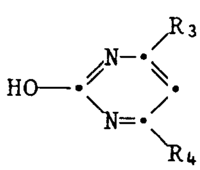

(V)

cyclisiert und

2.2 die OH-Gruppe in der erhaltenen Verbindung der Formel V weiter mit überschüssigem $POHal_3$ in Gegenwart oder Abwesenheit eines Lösungsmittels bei Temperaturen von 50 ° C bis 110 ° C, bevorzugt bei der Rückflusstemperatur des $POHal_3$, gegen Halogen austauscht

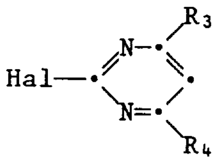

(VI)

wobei Hal in vorstehenden Formeln Halogen, besonders Chlor oder Brom, bedeutet, und

2.3 die erhaltene Verbindung der Formel VI weiter mit einer Anilinverbindung der Formel VII

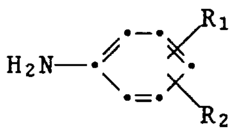

(VII)

je nach Verfahrensbedingungen entweder

a) in Gegenwart eines Protonenakzeptors, wie der überschüssigen Anilinverbindung der Formel VII oder einer anorganischen Base, mit oder ohne Lösungsmittel oder

b) in Gegenwart einer Säure in einem inerten Lösungsmittel bei jeweils Temperaturen von 60 °C bis 120 °C, bevorzugt 80 °C bis 100 °C, umsetzt oder

3. in einem zwei-stufigen Verfahren:

3.1 ein Guanidinsalz der Formel VIII

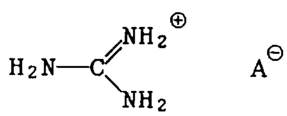

(VIII)

mit einem Diketon der Formel III

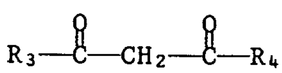

(III)

a) ohne Lösungsmittel bei Temperaturen von 100 °C bis 160 °C, bevorzugt 120 °C bis 150 °C, oder

b) in einem inerten Lösungsmittel bei Temperaturen von 30 °C bis 140 °C, bevorzugt 60 °C bis 120 °C,

zu einer Pyrimidinverbindung der Formel IX

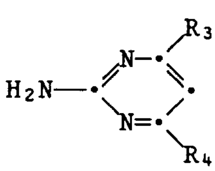

(IX)

cyclisiert und

3.2 die erhaltene Verbindung der Formel IX mit einer Verbindung der Formel X

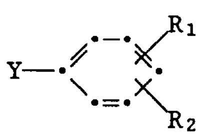

(X)

unter Abspaltung von HY in Gegenwart eines Protonenakzeptors in aprotischen Lösungsmitteln bei Temperaturen von 30 ° C bis 140 ° C, bevorzugt 60 ° C bis 120 ° C, umsetzt, wobei in den Formeln II bis X die Substituenten $R_1$ bis $R_4$ die unter Formel I angegebenen Bedeutungen besitzen, $A^{\ominus}$ ein Säureanion und Y Halogen darstellen, oder aber

4. in einem mehrstufigen Verfahren:

4.1

a) Thioharnstoff der Formel XI

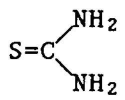

(XI)

mit einem Diketon der Formel III

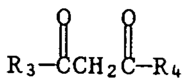

(III)

in Gegenwart einer Säure in einem inerten Lösungsmittel bei Temperaturen von 20 ° C bis 140 ° C, bevorzugt 20 ° C bis 60 ° C, zur Reaktion bringt und zu einer Pyrimidinverbindung der Formel

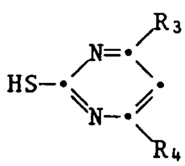

(XII)

cyclisiert und deren Alkali – oder Erdalkalisalz mit einer Verbindung der Formel XIII

$ZR_5$ (XIII)

wobei $R_5$ $C_1-C_8$ –Alkyl oder unsubstituiertes oder mit Halogen und/oder $C_1-C_4$ Alkyl substituiertes Benzyl und Z Halogen bedeuten, zu einer Pyrimidinverbindung der Formel XIV

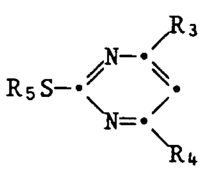

(XIV)

umsetzt, oder

b) ein Isothiuroniumsalz der Formel XV

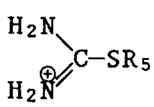

(XV)

mit einem Diketon der Formel III, bevorzugt in einem protischen Lösungsmittel, bei Temperaturen von 20 ° C bis 140 ° C, bevorzugt bei 20 ° C bis 80 ° C, zur Reaktion bringt und ebenfalls zu einer Pyrimidinverbindung der Formel XIV gelangt und

4.2 die erhaltene Verbindung der Formel XIV mit einem Oxydationsmittel, z.B. mit einer Persäure, zu der Pyrimidinverbindung der Formel XVI

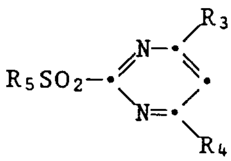

(XVI)

oxydiert und

4.3 die erhaltene Verbindung der Formel XVI mit einem Formylanilin der Formel XVII

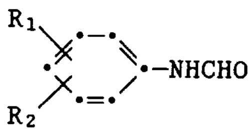

(XVII)

in einem inerten Lösungsmittel in Gegenwart einer Base als Protonenakzeptor bei Temperaturen zwischen −30 °C bis 120 °C zu einer Verbindung der Formel XVIII

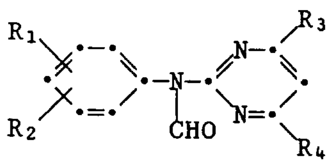

(XVIII)

umsetzt und

4.4 die erhaltene Verbindung der Formel XVIII einer Hydrolyse in Gegenwart einer Base, z.B. Alkalihydroxid, oder einer Säure, z.B. Halogenwasserstoffsäure oder Schwefelsäure, in Wasser oder wässerigen Lösungsmittelgemischen, wie wässerigen Alkoholen oder Dimethylformamid, bei Temperaturen von 10 °C bis 110 °C, bevorzugt 30 °C bis 60 °C, unterwirft, wobei in den Formeln XI bis XVIII die Substituenten $R_1$ bis $R_4$ die unter Formel I angegebenen Bedeutungen besitzen, und $A^{\ominus}$ ein Säureanion und Y Halogen darstellen.

Verbindungen der Formel I, in denen $R_3$ die $CH_2OH$-Gruppe darstellt, sind in speziellen Verfahren herstellbar, indem man

A1.1 das Guanidinsalz der Formel IIa

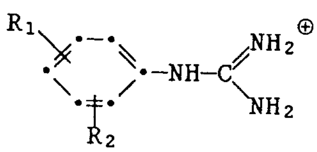

$A^{\ominus}$ (IIa)

oder das Guanidin der Formel IIb

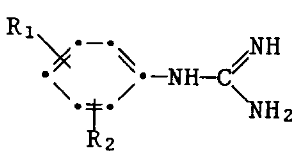

(IIb)

mit einem Keton der Formel XIX

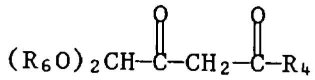

(XIX)

worin $R_6$ $C_1-C_4$-Alkyl bedeutet, in einem protischen Lösungsmittel oder ohne Lösungsmittel bei Temperaturen von 40 °C bis 160 °C, bevorzugt 60 °C bis 110 °C, zu einer Pyrimidinverbindung der Formel XX

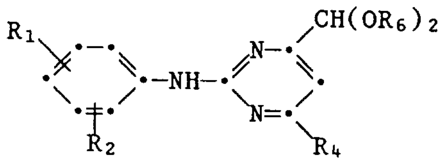

(XX)

umsetzt und

A1.2 das erhaltene Acetal der Formel XX in Gegenwart einer Säure, z.B. Halogenwasserstoffsäure oder Schwefelsäure, in Wasser oder wässerigen Lösungsmittelgemischen, z.B. mit Lösungsmitteln wie Alkoholen oder Dimethylformamid, bei Temperaturen von 20 °C bis 100 °C, bevorzugt 30 °C bis 60 °C, zum Pyrimidinaldehyd der Formel XXI

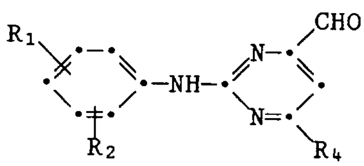

(XXI)

hydrolysiert und

A1.3 die erhaltene Verbindung der Formel XXI mit elementarem Wasserstoff unter Verwendung eines Katalysators hydriert oder mit einem Reduktionsmittel wie Natriumborhydrid zum entsprechenden Alkohol XXII reduziert

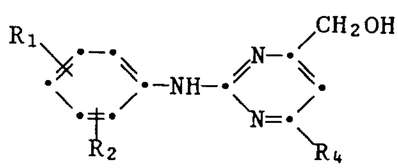

(XXII);

oder

A2.1 das Guanidinsalz der Formel IIa oder das Guanidin der Formel IIb mit einem Diketon der Formel XXIII

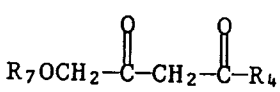

(XXIII)

worin $R_7$ unsubstituiertes oder mit Halogen oder $C_1-C_4$-Alkyl substituiertes Benzyl darstellt, in einem protischen Lösungsmittel oder ohne Lösungsmittel bei Temperaturen von 40 °C bis 160 °C, bevorzugt 60 °C bis 110 °C, zu einer Pyrimidinverbindung der Formel XXIV

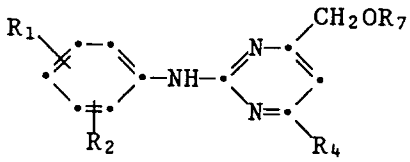

(XXIV)

umsetzt und in diesem durch

A2.2 Hydrierung in einem Lösungsmittel, vorzugsweise einem aprotischen Lösungsmittel, z.B. Dioxan oder Tetrahydrofuran, mit einem Katalysator wie Palladium-Kohle, bevorzugt Raney-Nickel, bei Temperaturen von 20°C bis 90°C, bevorzugt 50°C bis 90°C, den Rest $CH_2OR_7$ in den Rest $CH_2OH$ überführt; oder

A3.1 das Guanidinsalz der Formel IIa oder das Guanidin der Formel IIb mit einem Diketon der Formel XXV

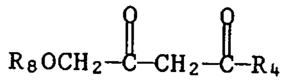

(XXV),

worin $R_8$ $C_1-C_6$-Alkyl, $C_3-C_6$-Alkenyl oder unsubstituiertes oder mit Halogen oder $C_1-C_4$-Alkyl substituiertes Benzyl darstellt, in einem protischen Lösungsmittel oder ohne Lösungsmittel bei Temperaturen von 40°C bis 160°C, bevorzugt 60°C bis 110°C zu einer Pyrimidinverbindung XXVI

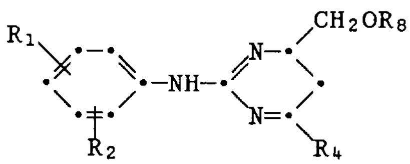

(XXVI)

umsetzt und

A3.2 mit der erhaltenen Verbindung der Formel XXVI eine Etherspaltung mit einer Halogenwasserstoffsäure, bevorzugt Bromwasserstoffsäure, oder einer Lewis-Säure wie Aluminiumhalogenid (z.B. $AlCl_3$) oder Borhalogenid $B(Hal)_3$ (z.B. $BBr_3$ oder $BCl_3$) in aprotischen Lösungsmitteln, z.B. Kohlenwasserstoffen, oder Halogenkohlenwasserstoffen, bei Temperaturen von −80°C bis 30°C, bevorzugt −70°C bis 20°C, durchführt.

Verbindungen der Formel I, in denen $R_3$ die $CH_2Hal$-Gruppe darstellt, sind herstellbar, indem man eine Verbindung der Formel XXII mit Phosphorhalogenid oder Thionylhalogenid in Gegenwart von tertiären Basen, z.B. Pyridin oder Triethylamin, in inerten Lösungsmitteln, bei Temperaturen von 0°C bis 110°C, bevorzugt von 0°C bis 80°C, umsetzt.

Verbindungen der Formel I, in denen $R_3$ die $CH_2F$-Gruppe bedeutet, lassen sich herstellen, indem man eine Verbindung der Formel XXVII

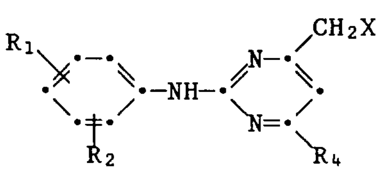

(XXVII)

worin X Chlor oder Brom bedeutet, mit Kaliumfluorid, bevorzugt lyophilisiertem Kaliumfluorid, in Gegenwart von katalytischen Mengen Cäsiumfluorid oder einem Kronenether, z.B. 18-Crown-6-ether, in aprotischen Lösungsmitteln wie Acetonitril, bei Temperaturen von 50°C bis 160°C in einem Druckautoklaven reagieren lässt.

Ein weiteres Verfahren zur Herstellung von Verbindungen der Formel I, in denen $R_3$ die $CH_2F$-Gruppe darstellt, besteht in der Fluorierung einer Verbindung der Formel XXII mit N,N-Diethylaminoschwefeltri-

fluorid (= DAST) in aprotischen Lösungsmitteln wie Dichlormethan, Chloroform, Tetrahydrofuran oder Dioxan, bei Temperaturen von 0° C bis 100° C, bevorzugt 10° C bis 50° C.

Auch in den vorstehenden Formeln XVIII bis XXVII haben $R_1$, $R_2$ und $R_4$ die für Formel I gegebene Bedeutung.

In den beschriebenen Verfahren kommen bei den Verbindungen der Formeln IIa und VIII für das Säureanion $A^{\ominus}$ beispielsweise folgende Salzreste in Betracht: Carbonat, Hydrogencarbonat, Nitrat, Halogenid, Sulfat oder Hydrogensulfat.

Im vorstehend beschriebenen Verfahren kommen bei der Verbindung der Formel XV für das Säureanion $A^{\ominus}$ beispielsweise folgende Salze in Betracht: Halogenid, Sulfat oder Hydrogensulfat.

Unter Halogenid sind jeweils Fluorid, Chlorid, Bromid oder Jodid, bevorzugt Bromid oder Chlorid, zu verstehen.

Als Säuren finden vornehmlich anorganische Säuren, wie z.B. Halogenwasserstoffsäuren, beispielsweise Fluorwasserstoffsäure, Chlorwasserstoffsäure oder Bromwasserstoffsäure, sowie Schwefelsäure, Phosphorsäure oder Salpetersäure Verwendung; jedoch können auch geeignete organische Säuren wie Essigsäure, Toluolsulfonsäure u.a. verwendet werden.

Als Protonenakzeptoren dienen z.B. anorganische oder organische Basen, wie beispielsweise Alkali- oder Erdalkaliverbindungen, z.B. die Hydroxide, Oxide oder Karbonate von Lithium, Natrium, Kalium, Magnesium, Calcium, Strontium und Barium oder auch Hydride wie z.B. Natriumhydrid. Als organische Basen seien beispielsweise tertiäre Amine wie Triethylamin, Triethylendiamin, Pyridin genannt.

In den vorstehend beschriebenen Verfahren können in Anpassung an die jeweiligen Reaktionsbedingungen beispielsweise folgende Lösungsmittel neben den z.T. genannten verwendet werden.

Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Chlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Dichlorbenzol, Dibrombenzol, Chlortoluol, Trichlortoluol; Ether, wie Ethylpropylether, Methyl-tert.-butylether, n-Butylethylether, Di-n-butylether, Di-isobutylether, Diisoamylether, Diisopropylether, Anisol, Cyclohexylmethylether, Diethylether, Ethylenglykoldimethylether, Tetrahydrofuran, Dioxan, Thioanisol, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, wie Heptan, Hexan, Octan, Nonan, Cymol, Benzinfraktionen innerhalb eines Siedepunktintervalles von 70° C bis 190° C, Cyclohexan, Methylcyclohexan, Dekalin, Petrolether, Ligroin, Trimethylpentan, wie 2,3,3-Trimethylpentan; Ester wie Ethylacetat, Acetessigester, Isobutylacetat; Amide, z.B. Formamid, Methylformamid, Dimethylformamid; Ketone, wie Aceton, Methylethylketon; Alkohole, insbesondere niedere aliphatische Alkohole, wie z.B. Methanol, Ethanol, n-Propanol, iso-Propanol sowie die Isomeren der Butanole; gegebenenfalls auch Wasser. Auch Gemische der genannten Lösungs- und Verdünnungsmittel kommen in Betracht.

Analoge Synthesemethoden zu den vorstehend beschriebenen Herstellungsverfahren sind in der Literatur publiziert.

Als Hinweise seien genannt:

Verfahren 1: A.Kreutzberger und J.Gillessen, J.Heterocyclic Chem.22, 101 (1985).

Verfahren 2, Stufe 2.1: O. Stark, Ber.Dtsch.Chem.Ges.42, 699 (1909); J. Hale, J.Am.Chem.Soc.36, 104 (1914); G.M. Kosolapoff, J.Org.Chem.26, 1895 (1961). Stufe 2.2: St. Angerstein, Ber.Dtsch.Chem.Ges.34, 3956 (1901); G.M. Kosolapoff, J.Org.Chem.26, 1895 (1961). Stufe 2.3: M.P.V. Boarland and J.F.W. McOmie, J.Chem.Soc. 1951, 1218; T.Matsukawa und K. Shirakuwa, J.Pharm.Soc.Japan 71, 933 (1951); Chem.Abstr.46, 4549 (1952).

Verfahren 3: A. Combes und C. Combes, Bull.Soc. Chem.(3), 7, 791 (1892); W.J. Hale und F.C. Vibrans, J.Am.Chem.Soc.40, 1046 (1918).

Die beschriebenen Herstellungsverfahren einschliesslich aller Teilschritte sind ein Bestandteil der vorliegenden Erfindung.

Folgende Verbindungen, die bei der Herstellung der Verbindungen der Formel I als Zwischenprodukte verwendet werden, sind neu und stellen einen Teil der vorliegenden Erfindung dar:

Verbindungen der Formel XXI

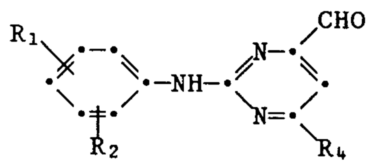

(XXI)

worin bedeuten:

$R_1$ und $R_2$ unabhängig voneinander Wasserstoff, Halogen, $C_1-C_3$-Alkyl, $C_1-C_2$-Halogenalkyl, $C_1-C_3$-Alkoxy oder $C_1-C_3$ Halogenalkoxy;

$R_4$ $C_3-C_6$-Cycloalkyl oder durch Methyl und/oder Halogen bis zu dreifach gleich oder verschieden substituiertes $C_3-C_6$-Cycloalkyl.

Ueberraschenderweise wurde nun gefunden, dass Verbindungen der Formel I ein für praktische Bedürfnisse sehr günstiges biozides Spektrum zur Bekämpfung von Insekten und phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel I sind z.B. gegen die den folgenden Klassen angehörenden phytopathogenen Pilze wirksam: Fungi imperfecti (insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula) und der Oomyceten (z.B. Phytophthora, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Verbindungen der Formel I sind darüberhinaus gegen Schadinsekten wirksam, z.B. gegen Getreide-Schädlinge, insbesondere Reisschädlinge.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk

(Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha, bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Besonders vorteilhafte applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, die man beispielsweise aus Sojabohnen gewinnen kann.

Als oberflächenaktive Verbindungen kommen je nach Art des zu formulierenden Wirkstoffs der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}-C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen.

Alkylarylsulfonate sind z.B. die Na-, Ca- oder Triethanolaminsalze der Dodecylbenzolsulfonsäure, der Dibutylnaphthalinsulfonsaure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind wasserlösliche 20 bis 250 Aethylenglykoläthergruppen und 10 bis 100 Propylenglykolethergruppen enthaltende Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,9 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

1. Herstellungsbeispiele

Beispiel 1.1 Herstellung von 2-Phenylamino-4-methyl-6-cyclopropyl-pyrimidin

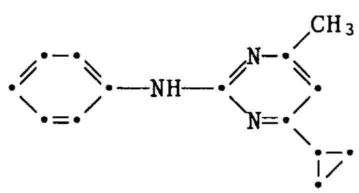

[Verb. Nr. 1.1]

10 g (51 mMol) Phenylguanidin-hydrogencarbonat und 9,7 g (77 mMol) 1-Cyclopropyl-1,3-butandion werden unter Rühren 6 Stunden auf 110°C erwärmt, wobei die einsetzende Kohlendioxidentwicklung mit fortschreitender Reaktionsdauer nachlässt. Nach dem Abkühlen auf Raumtemperatur wird die dunkelbraune Emulsion mit 50 ml Diethylether versetzt, zweimal mit je 20 ml Wasser gewaschen, über Natriumsulfat getrocknet, filtriert und das Lösungsmittel verdampft. Das zurückbleibende dunkelbraune Oel (= 13,1 g) wird säulenchromatographisch über Kieselgel (Diethylether/Toluol: 5/3) gereinigt. Nach Abdampfen des Laufmittelgemisches wird das braune Oel zur Kristallisation gebracht und aus Diethylether/Petrolether bei 30 - 50°C umkristallisiert. Es werden hellbraune Kristalle erhalten. Schmelzpunkt: 67 - 69°C; Ausbeute: 8,55 g (38 mMol) (= 74,5 % d. Th.).

Beispiel 1.2: Herstellung von 2 – Anilino – 4 – formyldiethylacetal – 6 – cyclopropyl – pyrimidin

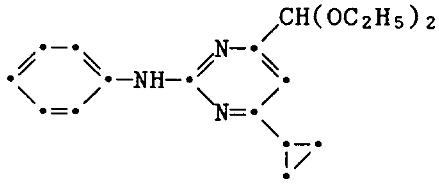

11,7 g (59,2 mMol) Phenylguanidin – hydrogencarbonat und 13,3 g (62,2 mMol) 1 – Cyclopropyl – 3 – formyldiethylacetal – 1,3 – propandion in 40 ml Ethanol werden unter Rühren 5 Stunden unter Rückfluss erhitzt, wobei die Kohlendioxidentwicklung mit fortschreitender Reaktionsdauer nachlässt. Nach dem Ab – kühlen auf Raumtemperatur wird die dunkelbraune Emulsion mit 80 ml Diethylether versetzt, zweimal mit je 30 ml Wasser gewaschen, über Natriumsulfat getrocknet, filtriert und das Lösungsmittel verdampft. Das zurünckbleibende dunkelbraune Oel (17 g) wird säulenchromatographisch über Kieselgel (Toluol/Ethylacetat:5/2) gereinigt. Nach dem Abdampfen des Laufmittelgemisches verbleibt ein rotbraunes Oel mit dem Brechungsindex $n_D^{25}$ :1,5815. Ausbeute: 15 g (48 mMol; 81,1 % d.Th.).

Beispiel 1.3 Herstellung von 2 – Anilino – 4 – formyl – 6 – cyclopropyl – pyrimidin

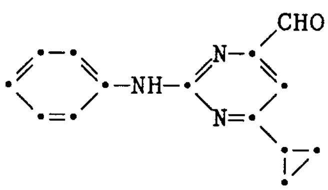

(Verb. Nr. 2.1)

12,3 g (39,3 mMol) 2 – Anilino – 4 – formyldiethylacetal – 6 – cyclopropyl – pyrimidin, 4 g (39,3 mMol) konz. Salzsäure und 75 ml Wasser werden unter intensivem Rühren 14 Stunden bei 50 ° C erwärmt und nach Zugabe von 2 g (19,6 mMol) konz. Salzsäure weitere 24 Stunden bei dieser Temperatur gerührt. Nach dem Abkühlen auf Raumtemperatur werden zu der beige gefärbten Suspension 50 ml Ethylacetat gegeben und mit 7 ml 30 %iger Natronlauge neutral gestellt. Die Ethylacetatlösung wird dann abgetrennt, über Natriumsulfat getrocknet, filtriert und das Lösungmittel verdampft. Zur Reinigung wird der bräunlich gefärbte Festkörper in Gegenwart von Aktivkohle aus 20 ml Isopropanol umkristallisiert. Die gelblichen Kristalle schmelzen bei 112 – 114 ° C. Ausbeute 7,9 g (33 mMol; 84 % d.Th.).

Beispiel 1.4: Herstellung von 2 – Anilino – 4 – hydroxymethyl – 6 – cyclopropyl – pyrimidin

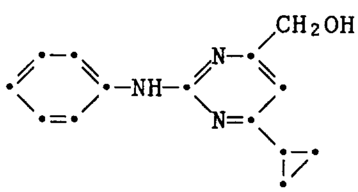

(Verb. Nr. 1.48)

a) Zu 14,1 g (59 mMol) 2 – Anilino – 4 – formyl – 6 – cyclopropyl – pyrimidin in 350 absolutem Methanol werden innerhalb von 15 Minuten unter Rühren bei Raumtemperatur 2,3 g (60 mMol) Natriumborhydrid portionenweise zugegeben, wobei sich das Reaktionsgemisch unter Wasserstoffentwicklung auf 28 ° C erwärmt. Nach 4 Stunden wird durch Zutropfen von 10 ml konz. Salzsäure angesäuert, 120 ml 10 %ige Natriumhydrogencarbonatlösung zugetropft und anschliessend mit 250 ml Wasser verdünnt. Der ausge – schiedene Niederschlag wird abfiltriert, getrocknet, in 600 ml Diethylether in der Wärme weitgehend gelöst, mit Aktivkohle behandelt und filtriert. Das klare Filtrat wird bis zum Auftreten einer Trübung

eingeengt, mit Petrolether verdünnt und das hellgelbe Kristallpulver abfiltriert;
Schmp. 123 – 125 ° C. Ausbeute: 10,8 g (44,8 mMol; 75,9 % d.Th.).
b) 5,9 g (23 mMol) 2-Anilino-4-methoxymethyl-6-cyclopropyl-pyrimidin, hergestellt aus Phenylguanidin und 1-Cyclopropyl-4-methoxy-1,3-butandion, werden in 200 ml Dichlormethan gelöst und auf –68 ° C gekühlt. Zu der lachsfarbenen Lösung werden unter intensivem Rühren 6,8 g (27 mMol) Bortribromid innerhalb einer halben Stunde langsam zugetropft, anschliessend das Kältebad entfernt und noch 2 Stunden bei Raumtemperatur gerührt. Nach der Zugabe von 150 g Eiswasser wird das ausgeschiedene Rohprodukt abfiltriert und aus Methanol unter Verwendung von Aktivkohle umkristallisiert. Die hellgelben Kristalle schmelzen bei 124 – 126 ° C. Ausbeute: 4,7 g (19,5 mMol; 84,7 % d.Th.).

Beispiel 1.5: Herstellung von 2-Phenylamino-4-brommethyl-6-cyclopropyl-pyrimidin

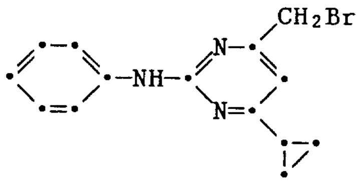

(Verb. Nr. 1.4)

Zu 12 g (50 mMol) 2-Phenylamino-4-hydroxymethyl-6-cyclopropyl-pyrimidin und 0,4 g (50 mMol) Pyridin in 350 ml Diethylether werden innerhalb einer halben Stunde 15,6 g (75 mMol) Thionylbromid in 50 ml Diethylether unter Rühren zugetropft. Nach 2-stündigem Rühren bei Raumtemperatur werden nochmals 0,4 g (50 mMol) Pyridin zugegeben und 5 Stunden unter Rückfluss erhitzt. Nach dem Abkühlen auf Raumtemperatur werden 200 ml Wasser zugegeben und durch Zutropfen von 140 ml gesättigter Natriumhydrogencarbonat-Lösung wird der pH-Wert 7 eingestellt. Nach dem Abtrennen wird die Diethyletherphase zweimal mit je 100 ml Wasser gewaschen, über Natriumsulfat getrocknet, filtriert und das Lösungmittel verdampft. Das zurückbleibende braune Oel wird säulenchromatographisch über Kieselgel (Toluol/Chloroform/Diethylether/Petrolether (Kp. 50 – 70 ° C): 5/3/1/1) gereinigt. Nach dem Abdampfen des Laufmittelgemisches wird das gelbe Oel mit Diethylether/Petrolether (Kp. 50 – 70 ° C) verdünnt und in der Kälte zur Kristallisation gebracht. Das gelbe Kristallpulver schmilzt zwischen 77,5 – 79,5 ° C. Ausbeute: 9,7 g (32 mMol; 64 % d.Th.).

Beispiel 1.6 Herstellung von 2-Phenylamino-4-fluormethyl-6-cyclopropyl-pyrimidin

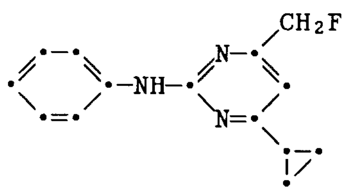

(Verb. Nr. 1.59)

a) 3,9 g (12,8 mMol) 2-Phenylamino-4-brommethyl-6-cyclopropyl-pyrimidin, 1,5 g (26 mMol) sprühgetrocknetes Kaliumfluorid und 0,3 g (1,13 mMol) 18-Crown-6-ether werden in 50 ml Acetonitril 40 Stunden unter Rückfluss erhitzt. Danach werden weiter 0,75 g (13 mMol) Kaliumfluorid zugegeben und 22 Stunden erwärmt. Zur Vervollständigung der Reaktion werden nochmals 0,75 g (13 mMol) sprühgetrocknetes Kaliumfluorid und 0,1 g (0,38 mMol) 18-Crown-6-ether hinzugefügt und weitere 24 Stunden unter Rückfluss erhitzt. Nach dem Abkühlen auf Raumtemperatur wird die Suspension mit 150 ml Diethylether versetzt, dreimal mit je 20 ml Wasser gewaschen, über Natriumsulfat getrocknet, filtriert und das Lösungsmittel verdampft. Das zurückbleibende braune Oel wird säulenchromatographisch über Kieselgel (Toluol/Chloroform/Diethylether/Petrolether (Kp. 50 – 70 ° C): 5/3/1/1) gereinigt. Nach dem Abdampfen des Laufmittelgemisches wird das gelbe Oel mit 10 ml Petrolether (Kp. 50 – 70 ° C) verdünnt und in der Kälte zur Kristallisation gebracht. Die gelben Kristalle schmelzen bei 48 – 52 ° C; Ausbeute: 2,1 g (8,6 mMol); 67,5 % d.Th.

b) Zu einer Suspension von 9,1 g (37,8 mMol) 2-Phenylamino-4-hydroxymethyl-6-cyclopropylpyrimidin in 80 ml Dichlormethan werden unter Rühren innerhalb 1 Stunde 6,1 g (37,8 mMol) Diethylaminoschwefeltrifluorid in 15 ml Dichlormethan langsam zugetropft. Nach der Zugabe von 50 ml Eiswasser werden 50 ml 10 %ige wässrige Natriumhydrogencarbonatlösung zugetropft. Nach beendeter Kohlendioxydentwicklung wird die organische Phase abgetrennt und die wässrige Phase zweimal mit je 20 ml Dichlormethan extrahiert. Die vereinigten Dichlormethanlösungen werden mit 15 ml Wasser gewaschen, über Natriumsulfat getrocknet, filtriert und das Lösungsmittel verdampft. Das zurückbleibende schwarze Oel wird säulenchromatographisch über Kieselgel (Toluol/Chloroform/Diethylether/Petrolether (Kp. 50 - 70°); 5/3/1/1) gereinigt. Nach dem Abdampfen des Laufmittelgemisches wird das gelbe Oel mit 20 ml Petrolether (Kp. 50 - 70°C) verdünnt und in der Kälte zur Kristallisation gebracht. Die gelblichen Kristalle schmelzen zwischen 50 - 52°C. Ausbeute: 4,9 g (20,1 mMol; 53 % d.Th.).

Beispiel 1.7: Herstellung von 2-Hydroxy-4-methyl-6-cyclopropyl-pyrimidin

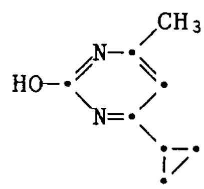

6 g (100 mMol) Harnstoff und 12,6 g (100 mMol) 1-Cyclopropyl-1,3-butandion werden bei Raumtemperatur in 35 ml Ethanol mit 15 ml konz. Salzsäure versetzt. Nach 10-tägigem Stehen bei Raumtemperatur wird am Rotationsverdampfer bei einer Badtemperatur von maximal 45°C eingeengt. Der Rückstand wird in 20 ml Ethanol gelöst, wobei sich nach kurzer Zeit das Hydrochlorid des Reaktionsproduktes abscheidet. Unter Rühren werden 20 ml Diethylether zugegeben, die ausgeschiedenen weissen Kristalle abfiltriert und mit einem Ethanol-Diethylethergemisch gewaschen und getrocknet. Durch Einengen des Filtrats und Umkristallisieren aus einem Ethanol/Diethylethergemisch:1/2wird ein weiterer Anteil Hydrochlorid gewonnen. Die weissen Kristalle schmelzen > 230°C. Ausbeute: Hydrochlorid 12,6 g (67,5 mMol; 67,5 % d.Th.).

Beispiel 1.8: Herstellung von 2-Chlor-4-methyl-6-cyclopropyl-pyrimidin

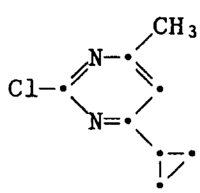

(Verb. Nr. 3.1)

52,8 g (0,24 Mol) 2-Hydroxy-4-methyl-6-cyclopropyl-pyrimidin-hydrochlorid werden unter Rühren bei Raumtemperatur in ein Gemisch von 100 ml (1,1 Mol) Phosphoroxychlorid und 117 g (0,79 Mol) Diethylanilin eingetragen, wobei die Temperatur auf 63°C ansteigt. Nach 2 stündigem Erhitzen auf 110° wird auf Raumtemperatur abgekühlt und das Reaktionsgemisch unter Rühren auf ein Eiswasser-Methylenchloridgemisch ausgetragen. Die organische Phase wird abgetrennt und mit gesättigter wässriger Natriumhydrogencarbonatlösung neutral gewaschen. Nach dem Abdampfen des Lösungsmittels werden 116,4 g Oel erhalten, das sich aus dem Reaktionsprodukt und Diethylanilin zusammensetzt. Die Abtrennung des Diäthylanilins und die Reinigung des rohen Reaktionsproduktes erfolgt durch Säulenchromatographie über Kieselgel (Hexan/Diethylacetat:3/1). Das nach einigen Tagen kristallisierende farblose Oel hat einen Brechungsindex $n_D^{25}$ :1.5419; Ausbeute: 35,7 g (0,21 Mol; 87,5 % d.Th.); Schmelzpunkt: 33 - 34°C.

Beispiel 1.9 Herstellung von 2-(m-Fluorphenylamino)-4-methyl-6-cyclopropyl-pyrimidin)

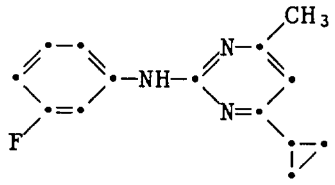

(Verb. Nr. 1.63)

Eine Lösung von 5,5 g (50 mMol) 3-Fluoranilin und 9,3 g (55 mMol) 2-Chlor-4-methyl-6-cyclopropyl-pyrimidin in 100 ml Ethanol wird unter Rühren mit 5 ml konz. Salzsäure auf pH 1 gebracht und anschliessend 18 Stunden unter Rückfluss erhitzt. Nach dem Abkühlen auf Raumtemperatur wird die braune Emulsion mit 10 ml 30 %igem Ammoniak alkalisch gestellt, auf 100 ml Eiswasser gegossen und zweimal mit je 150 ml Diethylether extrahiert. Die vereinigten Extrakte werden mit 50 ml Wasser gewaschen, über Natriumsulfat getrocknet, filtriert und das Lösungsmittel verdampft. Die zurückbleibenden gelblichen Kristalle werden durch Umkristallisation aus Diisopropylether/Petrolether (Kp. 50 − 70 ° C) gereinigt. Die weissen Kristalle schmelzen bei 87 − 89 ° C; Ausbeute 8,3 g (34 mMol; 68 % d.Th.).

Auf diese Art oder nach einer der weiter oben angegebenen Methoden lassen sich folgende Verbindungen der Formel I herstellen.

Tabelle 1: Verbindungen der Formel

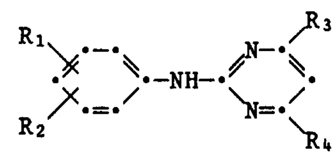

| Verb.Nr. | $R_1$ | $R_2$ | $R_3$ | $R_4$ | Physikalische Konstante |
| --- | --- | --- | --- | --- | --- |
| 1.1 | H | H | $CH_3$ | 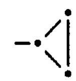 | Smp. 67-69°C |
| 1.2 | 2-Cl | H | $CH_3$ | 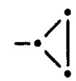 | |
| 1.3 | H | H | H | 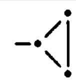 | Smp. 53-56°C |
| 1.4 | H | H | $-CH_2Br$ | 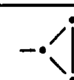 | Smp. 77,5-79,5°C |
| 1.5 | 3-Cl | H | $CH_3$ | 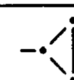 | Smp. 104-105°C |
| 1.6 | H | H | $-C_2H_5$ | 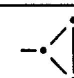 | Smp. 42-45°C |
| 1.7 | 4-Cl | H | $-CH_3$ | 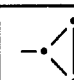 | Smp. 86-87°C |
| 1.8 | H | H | $-CH_2Br$ | 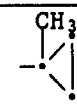 | |
| 1.9 | H | H | $-CH_2Cl$ | 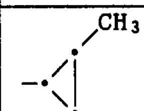 | Smp. 50-52°C |

Fortsetzung: Tabelle 1

| Verb.Nr. | $R_1$ | $R_2$ | $R_3$ | $R_4$ | Physikalische Konstante |
|---|---|---|---|---|---|
| 1.10 | $4-CH_3$ | H | $-CH_3$ | 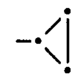 | Smp. 53-56°C |
| 1.11 | H | H | $-CF_3$ | 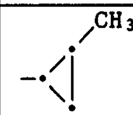 | |
| 1.12 | H | H | $-C_3H_7-n$ | 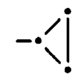 | Smp. 44-46°C |
| 1.13 | H | H | $-CH_2OH$ | 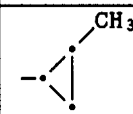 | Smp. 111-113°C |
| 1.14 | H | H | $-CH_3$ | 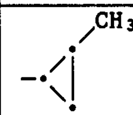 | Smp. 73-74°C |
| 1.15 | $4-OCH_3$ | H | $CH_3$ | 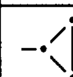 | Smp. 48-50°C |
| 1.16 | H | H | $-CH_2CH_2OH$ | 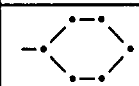 | |
| 1.17 | H | H | $-CH_2Br$ | 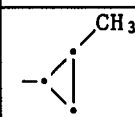 | |
| 1.18 | H | H | $-C_4H_9-n$ | 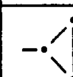 | dunkelbraunes Oel $n_D^{24}$: 1.5992 |
| 1.19 | H | H | $-CH_2OH$ | 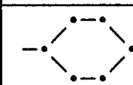 | |

Fortsetzung: Tabelle 1

| Verb.Nr. | $R_1$ | $R_2$ | $R_3$ | $R_4$ | Physikalische Konstante |
|---|---|---|---|---|---|
| 1.20 | $4-OC_2H_5$ | H | $CH_3$ | 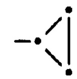 | Smp. 33-36°C |
| 1.21 | H | H | H | 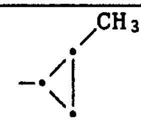 | |
| 1.22 | H | H | $-CH_2Br$ | 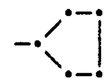 | |
| 1.23 | H | H | $-CH_2Br$ | 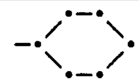 | |
| 1.24 | H | H | H | 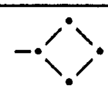 | |
| 1.25 | H | H | $-C_4H_9$ sek. | 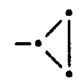 | Oel $n_D^{24}$: 1.6002 |
| 1.26 | H | H | $-CH_2OH$ | 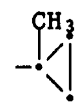 | |
| 1.27 | 4-Br | H | $-CH_3$ | 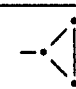 | Smp. 94-95°C |
| 1.28 | H | H | $-CH_3$ | 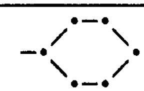 | Smp. 97-98°C |
| 1.29 | H | H | $-CF_3$ | 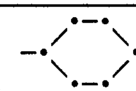 | |

**Fortsetzung:** Tabelle 1

| Verb.Nr. | $R_1$ | $R_2$ | $R_3$ | $R_4$ | Physikalische Konstante |
|---|---|---|---|---|---|
| 1.30 | H | H | $-C_4H_9$ tert. | 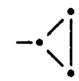 | |
| 1.31 | H | H | $-CH_3$ | 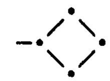 | Smp. 50-52°C |
| 1.32 | H | H | $-CF_3$ | 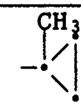 | |
| 1.33 | 4-F | H | $-CH_3$ | 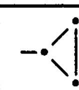 | Smp. 89-91°C |
| 1.34 | H | H | H | 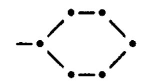 | |
| 1.35 | H | H | $-CH_2Cl$ | 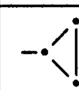 | Smp. 55-57°C |
| 1.36 | H | H | $-CF_3$ | 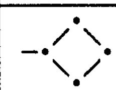 | |
| 1.37 | 4-$OCHF_2$ | H | $-CH_3$ | 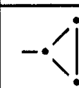 | Oel $n_D^{25}$:1.5763 |
| 1.38 | H | H | $-C_2H_5$ | 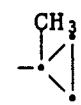 | |
| 1.39 | H | H | $-CHCl_2$ | 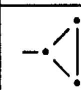 | Smp. 56-58°C |

Fortsetzung: Tabelle 1

| Verb.Nr. | $R_1$ | $R_2$ | $R_3$ | $R_4$ | Physikalische Konstante |
|---|---|---|---|---|---|
| 1.40 | 3-Cl | 5-Cl | $-CH_3$ | 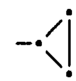 | |
| 1.41 | H | H | $-CHCl_2$ | 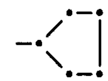 | |
| 1.42 | H | H | $-CH_3$ | 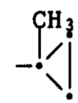 | Smp. 63-65°C |
| 1.43 | H | H | $-CH_2OH$ | 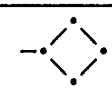 | |
| 1.44 | $3-OC_2H_5$ | $4-OC_2H_5$ | $-CH_3$ | 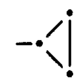 | Oel $n_D^{25}$: 1.5498 |
| 1.45 | H | H | $-CF_3$ | 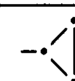 | Smp. 66-69°C |
| 1.46 | $4-OCF_3$ | H | $-CH_3$ | 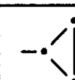 | |
| 1.47 | H | H | $-CH_2OH$ | 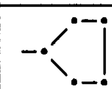 | |
| 1.48 | H | H | $-CH_2OH$ | 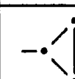 | Smp. 123-125°C |
| 1.49 | $3-CF_3$ | 4-Cl | $-CH_3$ | 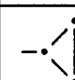 | Smp. 128-130°C |

Fortsetzung: Tabelle 1

| Verb.Nr. | $R_1$ | $R_2$ | $R_3$ | $R_4$ | Physikalische Konstante |
|---|---|---|---|---|---|
| 1.50 | H | H | H | 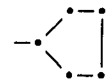 | |
| 1.51 | H | H | H | 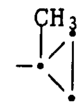 | |
| 1.52 | H | H | $-CH_2CH_2OH$ | 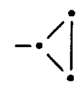 | |
| 1.53 | 3-Cl | 4-Cl | $-CH_3$ | 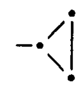 | Smp. 85-87°C |
| 1.54 | H | H | $-CH_3$ | 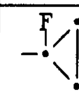 | Smp. 73-74°C |
| 1.55 | 2-F | H | $-CH_3$ | 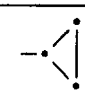 | |
| 1.56 | H | H | $-CH_3$ | 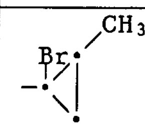 | |
| 1.57 | H | H | H | 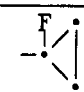 | |

<u>Fortsetzung:</u> Tabelle 1

| Verb.Nr. | $R_1$ | $R_2$ | $R_3$ | $R_4$ | Physikalische Konstante |
|---|---|---|---|---|---|
| 1.58 | H | H | $-CH_3$ | 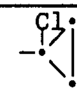 | Smp. 58-61°C |
| 1.59 | H | H | $-CH_2F$ | 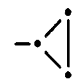 | Smp. 48-52°C |
| 1.60 | 3-Cl | $4-CH_3$ | $-CH_3$ | 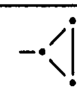 | |
| 1.61 | H | H | H | 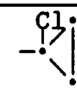 | |
| 1.62 | H | H | $-CH_3$ | 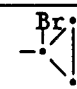 | |
| 1.63 | 3-F | H | $-CH_3$ | 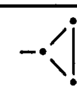 | Smp. 87-89°C |
| 1.64 | H | H | $-CH_3$ | 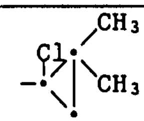 | |
| 1.65 | $2-CH_3$ | 3-Cl | $-CH_3$ | 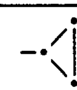 | |
| 1.66 | H | H | $-CH_3$ | 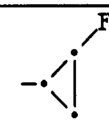 | Smp. 81-84°C |
| 1.67 | H | H | $-CH_2F$ | 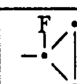 | Smp. 63-65°C |

Fortsetzung: Tabelle 1

| Verb.Nr. | $R_1$ | $R_2$ | $R_3$ | $R_4$ | Physikalische Konstante |
|---|---|---|---|---|---|
| 1.68 | 2-Cl | 5-$CH_3$ | -$CH_3$ | 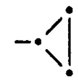 | |
| 1.69 | H | H | -$CH_3$ | 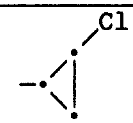 | Smp. 67-69°C |
| 1.70 | 2-Br | H | -$CH_3$ | 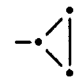 | |
| 1.71 | 2-$CH_3$ | 4-Cl | -$CH_3$ | 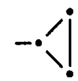 | |
| 1.72 | H | H | -$CH_3$ | 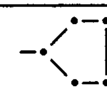 | Smp. 64-66°C |
| 1.73 | 2-Cl | 6-$CH_3$ | -$CH_3$ | 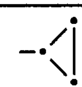 | |
| 1.74 | H | H | -$CH_2F$ | 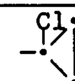 | Smp. 43-45°C |
| 1.75 | H | H | -$CH_2F$ | 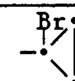 | |
| 1.76 | 3-Br | H | -$CH_3$ | 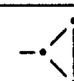 | |
| 1.77 | H | H | 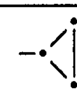 | 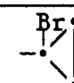 | |

Fortsetzung: Tabelle 1

| Verb.Nr. | $R_1$ | $R_2$ | $R_3$ | $R_4$ | Physikalische Konstante |
|---|---|---|---|---|---|
| 1.78 | H | H | $-CH_3$ | 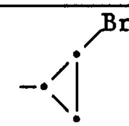 | Smp. 51-53°C |
| 1.79 | 2-Cl | 4-$CH_3$ | $-CH_3$ | 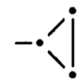 | |
| 1.80 | H | H | 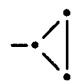 | 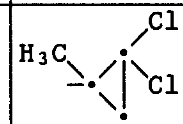 | Oel $n_D^{24}$: 1.6101 |
| 1.81 | H | H | $-CH_2F$ | 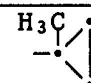 | |
| 1.82 | 3-Cl | 4-F | $-CH_3$ | 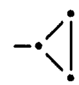 | |
| 1.83 | H | H | $-C_3H_7$-i | 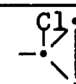 | |
| 1.84 | H | H | $-CH_3$ | 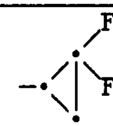 | Smp. 81-84°C |
| 1.85 | H | H | $-C_3H_7$-i | 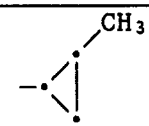 | |

Fortsetzung: Tabelle 1

| Verb.Nr. | $R_1$ | $R_2$ | $R_3$ | $R_4$ | Physikalische Konstante |
|---|---|---|---|---|---|
| 1.86 | 4-J | H | $-CH_3$ | 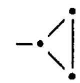 | |
| 1.87 | H | H | $-CH_2F$ | 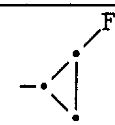 | Smp. 63-65°C |
| 1.88 | H | H | $-C_4H_9$-n | 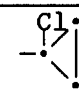 | |
| 1.89 | 2-$CH_3$ | H | $-CH_3$ | 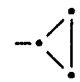 | |
| 1.90 | H | H | $-C_3H_7$-i | 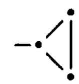 | Oel $n_D^{24}$: 1.6074 |
| 1.91 | H | H | $-CH_3$ | 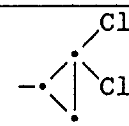 | Smp. 65-68°C |
| 1.92 | 2-$CH_3$ | 5-Cl | $-CH_3$ | 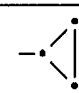 | |
| 1.93 | H | H | $-CH_3$ | 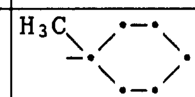 | |
| 1.94 | H | H | $-CH_2F$ | 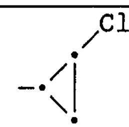 | Smp. 48-50°C |

Fortsetzung: Tabelle 1

| Verb.Nr. | $R_1$ | $R_2$ | $R_3$ | $R_4$ | Physikalische Konstante |
|---|---|---|---|---|---|
| 1.95 | 2-$OCH_3$ | 5-Cl | $-CH_3$ | 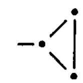 | |
| 1.96 | 3-Cl | 4-$OCH_3$ | $-CH_3$ | 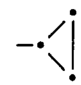 | |
| 1.97 | H | H | $-CH_2CH_2OH$ | 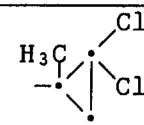 | |
| 1.98 | H | H | $-CH_3$ | 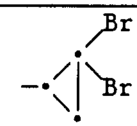 | |
| 1.99 | 2-Br | 4-Br | $-CH_3$ | 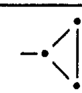 | |
| 1.100 | H | H | $-CH_2F$ | 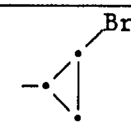 | Smp. 38-41°C |
| 1.101 | 3-$CH_3$ | H | $-CH_3$ | 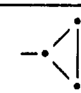 | |
| 1.102 | H | H | $-C_4H_9$-n | 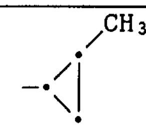 | |

Fortsetzung: Tabelle 1

| Verb.Nr. | $R_1$ | $R_2$ | $R_3$ | $R_4$ | Physikalische Konstante |
|---|---|---|---|---|---|
| 1.103 | 3-$CH_3$ | 4-Br | $-CH_3$ | 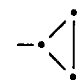 | |
| 1.104 | H | H | $-C_3H_7$-n | 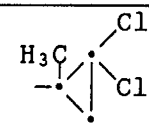 | |
| 1.105 | H | H | $-CH_3$ | 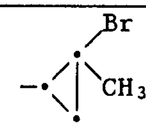 | |
| 1.106 | H | H | $-CH_2CH_2OH$ | 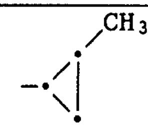 | |
| 1.107 | 2-$C_2H_5$ | H | $-CH_3$ | 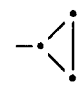 | |
| 1.108 | H | H | $-CH_2F$ | 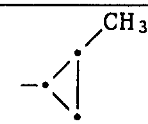 | Smp. 55-57°C |
| 1.109 | H | H | H | 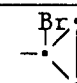 | |
| 1.110 | H | H | $-C_3H_7$-n | 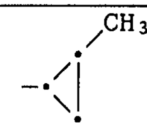 | |

Fortsetzung: Tabelle 1

| Verb.Nr. | $R_1$ | $R_2$ | $R_3$ | $R_4$ | Physikalische Konstante |
|---|---|---|---|---|---|
| 1.111 | $3-C_2H_5$ | H | $-CH_3$ | 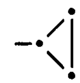 | |
| 1.112 | H | H | $-CH_2F$ | 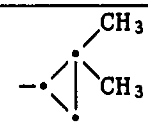 | |
| 1.113 | H | H | $-CH_3$ | 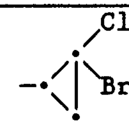 | Smp. 83-85°C |
| 1.114 | 2-Br | 5-Br | $-CH_3$ | 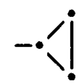 | |
| 1.115 | $2-CH_3$ | 4-Br | $-CH_3$ | 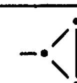 | |
| 1.116 | H | H | $-C_4H_9$-sek. | 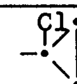 | |
| 1.117 | $2-CH_3$ | 5-F | $-CH_3$ | 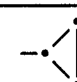 | |
| 1.118 | $4-C_2H_5$ | H | $-CH_3$ | 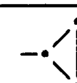 | |
| 1.119 | H | H | H | 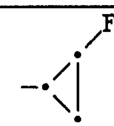 | |

Fortsetzung: Tabelle 1

| Verb.Nr. | $R_1$ | $R_2$ | $R_3$ | $R_4$ | Physikalische Konstante |
|---|---|---|---|---|---|
| 1.120 | H | H | $-CH_3$ | 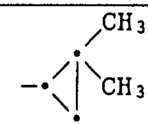 | Smp. 51-54°C |
| 1.121 | 2-Br | 4-$CH_3$ | $-CH_3$ | 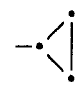 | |
| 1.122 | H | H | $-CH_2F$ | 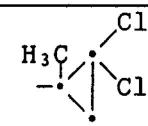 | |
| 1.123 | H | H | $-CF_3$ | 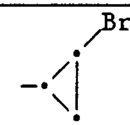 | |
| 1.124 | H | H | $-CF_3$ | 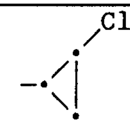 | |
| 1.125 | 2-$C_3H_7$-i | H | $-CH_3$ | 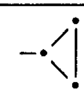 | |
| 1.126 | H | H | $-CH_2F$ | 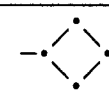 | Smp. 44-47°C |
| 1.127 | H | H | $-CH_3$ | 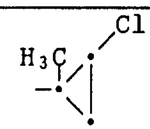 | |

Fortsetzung: Tabelle 1

| Verb.Nr. | $R_1$ | $R_2$ | $R_3$ | $R_4$ | Physikalische Konstante |
|---|---|---|---|---|---|
| 1.128 | H | H | 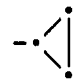 | 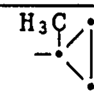 | Smp. 54-56°C |
| 1.129 | 2-Cl | 4-Br | $-CH_3$ | 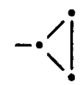 | |
| 1.130 | H | H | $-C_4H_9$-sek. | 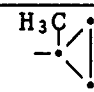 | |
| 1.131 | H | H | $-C_2H_5$ | 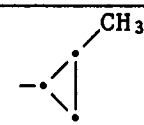 | Smp. 57-59°C |
| 1.132 | 4-$C_3H_7$-i | H | $-CH_3$ | 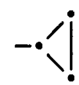 | |
| 1.133 | 2-$OCH_3$ | 5-$CH_3$ | $-CH_3$ | 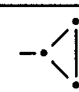 | |
| 1.134 | H | H | $-CH_3$ | 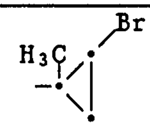 | |
| 1.135 | H | H | $-CH_2Cl$ | 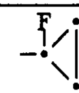 | |
| 1.136 | 3-$CF_3$ | 5-$CF_3$ | $-CH_3$ | 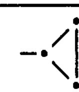 | |

Fortsetzung: Tabelle 1

| Verb.Nr. | $R_1$ | $R_2$ | $R_3$ | $R_4$ | Physikalische Konstante |
|---|---|---|---|---|---|
| 1.137 | H | H | $-CF_3$ | 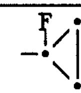 | |
| 1.138 | H | H | $-C_2H_5$ | 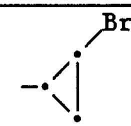 | |
| 1.139 | $2-CF_3$ | H | $-CH_3$ | 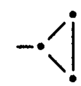 | Smp. 56-58°C |
| 1.140 | H | H | H | 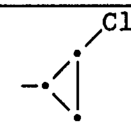 | |
| 1.141 | H | H | $-CH_3$ | 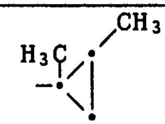 | |
| 1.142 | H | H | $-CH_2Cl$ | 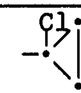 | |
| 1.143 | 2-Cl | 3-Cl | $-CH_3$ | 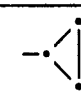 | |
| 1.144 | H | H | $-C_4H_9$-tert. | 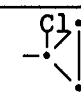 | |

Fortsetzung: Tabelle 1

| Verb.Nr. | $R_1$ | $R_2$ | $R_3$ | $R_4$ | Physikalische Konstante |
|---|---|---|---|---|---|
| 1.145 | H | H | $-C_2H_5$ | 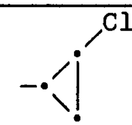 | Smp. 55-60°C |
| 1.146 | $2-CF_3$ | 4-Cl | $-CH_3$ | 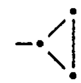 | |
| 1.147 | 2-Cl | 4-Cl | $-CH_3$ | 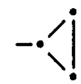 | |
| 1.148 | H | H | $-CH_3$ | 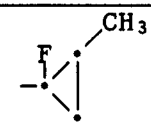 | |
| 1.149 | H | H | $-CH_2Cl$ | 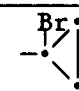 | |
| 1.150 | $3-CF_3$ | H | $-CH_3$ | 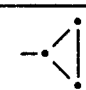 | Smp. 81-83°C |
| 1.151 | H | H | $-C_4H_9$-tert. | 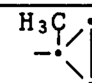 | |
| 1.152 | $4-CF_3$ | H | $-CH_3$ | 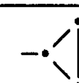 | Smp. 60-62°C |
| 1.153 | 2-Cl | $5-CF_3$ | $-CH_3$ | 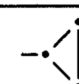 | |

Fortsetzung: Tabelle 1

| Verb.Nr. | $R_1$ | $R_2$ | $R_3$ | $R_4$ | Physikalische Konstante |
|---|---|---|---|---|---|
| 1.154 | H | H | $-CH_2Cl$ | 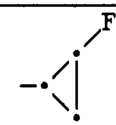 | Smp. 63-66°C |
| 1.155 | H | H | $-CH_3$ | 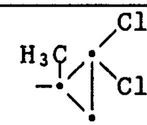 | Smp. 99-109°C |
| 1.156 | 2-Cl | 5-Cl | $-CH_3$ | 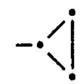 | |
| 1.157 | $4-OC_3H_7-i$ | H | $-CH_3$ | 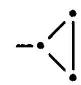 | |
| 1.158 | H | H | $-C_2H_5$ | 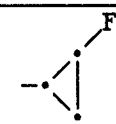 | Smp. 58-61°C |
| 1.159 | 2-Cl | 6-Cl | $-CH_3$ | 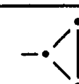 | |
| 1.160 | H | H | $-C_2H_5$ | 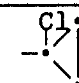 | |
| 1.161 | H | H | $-CH_3$ | 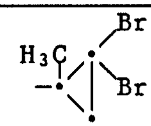 | |

Fortsetzung: Tabelle 1

| Verb.Nr. | $R_1$ | $R_2$ | $R_3$ | $R_4$ | Physikalische Konstante |
|---|---|---|---|---|---|
| 1.162 | H | H | $-CH_2Cl$ | 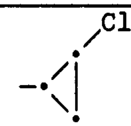 | Smp. 55-57°C |
| 1.163 | 2-$OCH_3$ | H | $-CH_3$ | 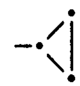 | |
| 1.164 | H | H | $-CH_2CH_2CH_2Cl$ | 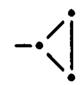 | |
| 1.165 | H | H | H | 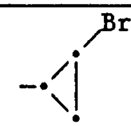 | |
| 1.166 | 2-F | 3-F | $-CH_3$ | 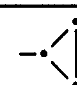 | |
| 1.167 | H | H | $-C_2H_5$ | 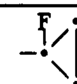 | |
| 1.168 | H | H | $-CH_3$ | 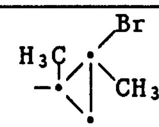 | |
| 1.169 | H | H | $-CH_2Cl$ | 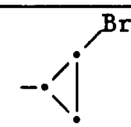 | |

Fortsetzung: Tabelle 1

| Verb.Nr. | $R_1$ | $R_2$ | $R_3$ | $R_4$ | Physikalische Konstante |
|---|---|---|---|---|---|
| 1.170 | $3-OCH_3$ | H | $-CH_3$ | 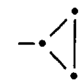 | Smp. 47-50°C |
| 1.171 | H | H | $-CH_2Cl$ | 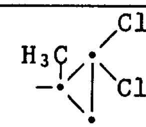 | |
| 1.172 | $2-CH_3$ | $4-OCH_3$ | $-CH_3$ | 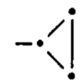 | |
| 1.173 | 2-F | 4-F | $-CH_3$ | 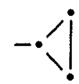 | |
| 1.174 | H | H | $-CH_2CH_2CH_2Cl$ | 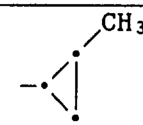 | |
| 1.175 | H | H | $-CH_3$ | 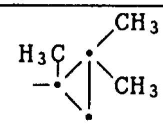 | |
| 1.176 | H | H | $-CH_2Cl$ | 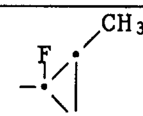 | |
| 1.177 | $2-OCHF_2$ | H | $-CH_3$ | 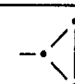 | |

**Fortsetzung:** Tabelle 1

| Verb.Nr. | $R_1$ | $R_2$ | $R_3$ | $R_4$ | Physikalische Konstante |
|---|---|---|---|---|---|
| 1.178 | H | H | $-CH_2OH$ | 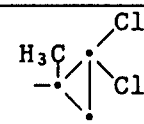 | |
| 1.179 | H | H | H | 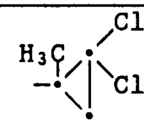 | |
| 1.180 | 2-F | 5-F | $-CH_3$ | 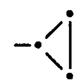 | Oel |
| 1.181 | H | H | $-CH_2OH$ | 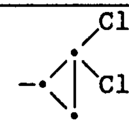 | |
| 1.182 | H | H | $-CH_3$ | 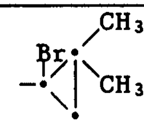 | |
| 1.183 | H | H | $-CHCl_2$ | 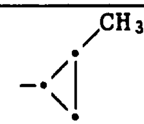 | |
| 1.184 | $4-OCF_2CHF_2$ | H | $-CH_3$ | 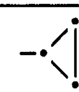 | |
| 1.185 | H | H | $-CH_2Cl$ | 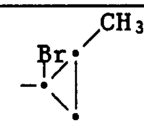 | |

Fortsetzung: Tabelle 1

| Verb.Nr. | $R_1$ | $R_2$ | $R_3$ | $R_4$ | Physikalische Konstante |
|---|---|---|---|---|---|
| 1.186 | H | H | H | 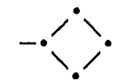 | |
| 1.187 | H | H | 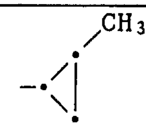 | 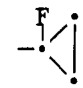 | Oel |
| 1.188 | H | H | 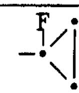 | 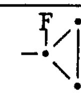 | |
| 1.189 | H | H | $-CH_3$ | 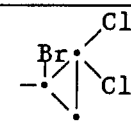 | |
| 1.190 | $4-OCF_2CHClF$ | H | $-CH_3$ | 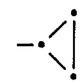 | |
| 1.191 | H | H | $-CH_2Cl$ | 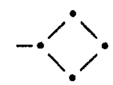 | |
| 1.192 | 2-F | 6-F | $-CH_3$ | 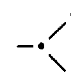 | |
| 1.193 | H | H | 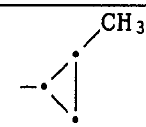 | 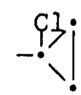 | |
| 1.194 | $4-OC_3H_7-n$ | H | $-CH_3$ | 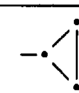 | |

Fortsetzung: Tabelle 1

| Verb.Nr. | $R_1$ | $R_2$ | $R_3$ | $R_4$ | Physikalische Konstante |
|---|---|---|---|---|---|
| 1.195 | H | H | 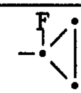 | 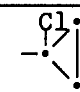 | |
| 1.196 | H | H | $-CH_3$ | 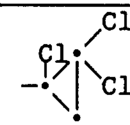 | |
| 1.197 | H | H | 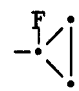 | 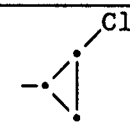 | |
| 1.198 | $4-OCF_2CHCl_2$ | H | $-CH_3$ | 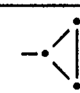 | |
| 1.199 | H | H | 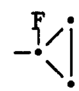 | 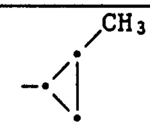 | |
| 1.200 | 3-F | 4-F | $-CH_3$ | 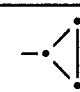 | Oel |
| 1.201 | H | H | $-CClF_2$ | 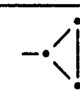 | Smp. 68-70°C |
| 1.202 | $3-OC_2H_5$ | H | $-CH_3$ | 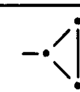 | |

Fortsetzung: Tabelle 1

| Verb.Nr. | $R_1$ | $R_2$ | $R_3$ | $R_4$ | Physikalische Konstante |
|---|---|---|---|---|---|
| 1.203 | H | H | $-CH_3$ | 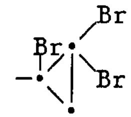 | |
| 1.204 | H | H | $-CH_2Br$ | 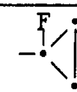 | |
| 1.205 | $4-OCF_2CFCl_2$ | H | $-CH_3$ | 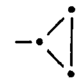 | |
| 1.206 | $2-OCH_3$ | $5-OCH_3$ | $-CH_3$ | 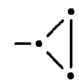 | |
| 1.207 | H | H | $-CH_2OH$ | 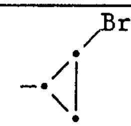 | |
| 1.208 | H | H | $-CClF_2$ | 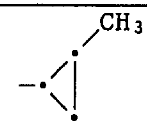 | |
| 1.209 | H | H | $-CH_2Br$ | 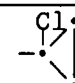 | |
| 1.210 | $2-OCH_3$ | $4-OCH_3$ | $-CH_3$ | 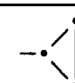 | |

Fortsetzung: Tabelle 1

| Verb.Nr. | $R_1$ | $R_2$ | $R_3$ | $R_4$ | Physikalische Konstante |
|---|---|---|---|---|---|
| 1.211 | H | H | 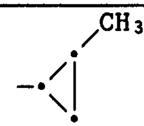 | 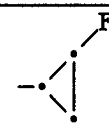 | Oel |
| 1.212 | H | H | $-CH_2Br$ | 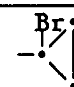 | |
| 1.213 | H | H | 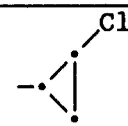 | 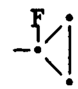 | |
| 1.214 | H | H | $-CH_2OH$ | 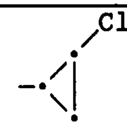 | |
| 1.215 | H | H | 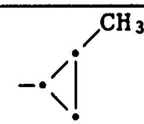 | 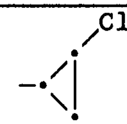 | |
| 1.216 | 3-$OCH_3$ | 5-$OCH_3$ | $-CH_3$ | 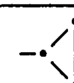 | |
| 1.217 | H | H | $-CH_2Br$ | 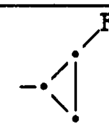 | |
| 1.218 | H | H | $CH_3$<br>$-CH_2CHCH_3$ | 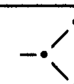 | Smp. 44-46°C |

Fortsetzung: Tabelle 1

| Verb.Nr. | $R_1$ | $R_2$ | $R_3$ | $R_4$ | Physikalische Konstante |
|---|---|---|---|---|---|
| 1.219 | $2-CH_3$ | $3-CH_3$ | $-CH_3$ | 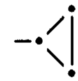 | |
| 1.220 | H | H | $-CH_2OH$ | 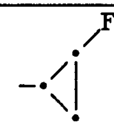 | |
| 1.221 | H | H | 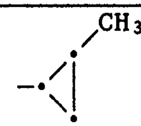 | 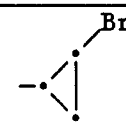 | |
| 1.222 | H | H | $CH_3$<br>$-CH_2CHCH_3$ | 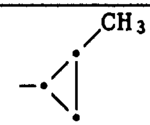 | |
| 1.223 | $2-CH_3$ | $4-CH_3$ | $-CH_3$ | 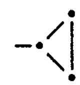 | |
| 1.224 | H | H | $-CH_2Br$ | 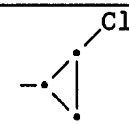 | |
| 1.225 | H | H | 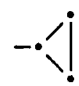 | 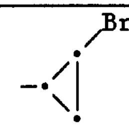 | |
| 1.226 | $2-CH_3$ | $5-CH_3$ | $-CH_3$ | 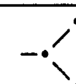 | |

Fortsetzung: Tabelle 1

| Verb.Nr. | $R_1$ | $R_2$ | $R_3$ | $R_4$ | Physikalische Konstante |
|---|---|---|---|---|---|
| 1.227 | H | H | $-CH_2OH$ | 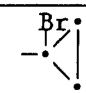 | |
| 1.228 | H | H | $-CF_2CF_3$ | 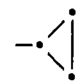 | Smp. 50-52°C |
| 1.229 | H | H | $-CH_2OH$ | 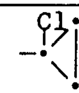 | |
| 1.230 | H | H | 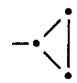 | 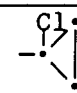 | |
| 1.231 | $2-CH_3$ | $6-CH_3$ | $-CH_3$ | 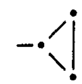 | |
| 1.232 | H | H | $-CH_2Br$ | 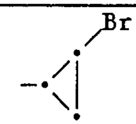 | |
| 1.233 | H | H | $-CF_2CF_3$ | 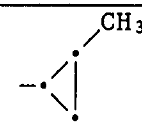 | |
| 1.234 | H | H | 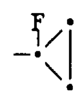 | 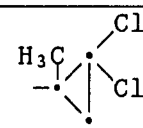 | |

Fortsetzung: Tabelle 1

| Verb.Nr. | $R_1$ | $R_2$ | $R_3$ | $R_4$ | Physikalische Konstante |
|---|---|---|---|---|---|
| 1.235 | H | H | 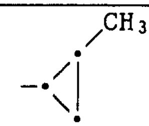 | 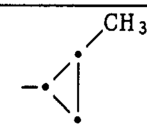 | Smp. 58-60°C |
| 1.236 | H | H | 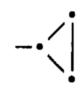 | 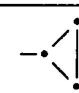 | Smp. 75-77°C |
| 1.237 | $3-CH_3$ | $4-CH_3$ | $-CH_3$ | 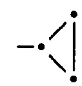 | |
| 1.238 | H | H | $-CH_2OH$ | 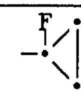 | Oel |
| 1.239 | H | H | $-CH_2Br$ | 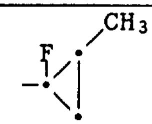 | |
| 1.240 | H | H | 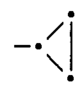 | 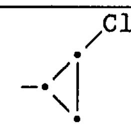 | |
| 1.241 | H | H | 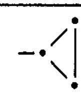 | 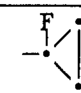 | |
| 1.242 | H | H | 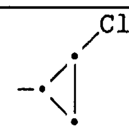 | 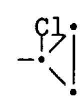 | |

Fortsetzung: Tabelle 1

| Verb.Nr. | $R_1$ | $R_2$ | $R_3$ | $R_4$ | Physikalische Konstante |
|---|---|---|---|---|---|
| 1.243 | H | H | $-CH_2Br$ | 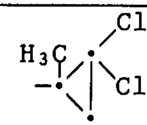 | |
| 1.244 | H | H | 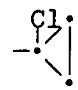 | 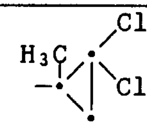 | |
| 1.245 | 3-$CH_3$ | 5-$CH_3$ | $-CH_3$ | 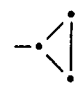 | |
| 1.246 | H | H | 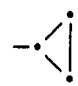 | 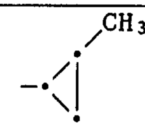 | Oel $n_D^{25}$: 1,6232 |
| 1.247 | 3-F | 4-$CH_3$ | $-CH_3$ | 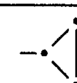 | |
| 1.248 | H | H | 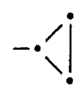 | 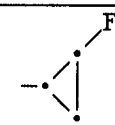 | |
| 1.249 | H | H | $-CH_2Br$ | 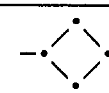 | |

Fortsetzung: Tabelle 1

| Verb.Nr. | $R_1$ | $R_2$ | $R_3$ | $R_4$ | Physikalische Konstante |
|---|---|---|---|---|---|
| 1.250 | $2-OCHF_2$ | $4-CH_3$ | $-CH_3$ | 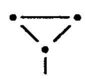 | Smp. 85-87°C |
| 1.251 | 3-Cl | $4-OCHF_2$ | $-CH_3$ | 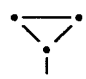 | $n_D^{25}$ 1.5898 |
| 1.252 | $3-OCH_3$ | $4-OCH_3$ | $-CH_3$ | 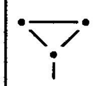 | Smp. 74-76°C |
| 1.253 | H | H | $-CH_3$ | 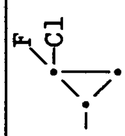 | Smp. 97-99°C |

In den folgenden Tabellen 2, 3 und 4 werden beispielhaft Zwischenprodukte vorliegender Erfindung genannt.

Tabelle 2: Verbindungen der Formel

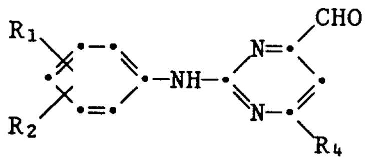

| Verb. Nr. | $R_1$ | $R_2$ | $R_4$ | Physikalische Konstante |
|---|---|---|---|---|
| 2.1 | H | H | 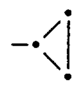 | Smp. 112-114°C |
| 2.2 | H | H | 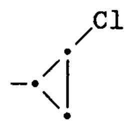 | Smp. 123-127°C |
| 2.3 | H | H | 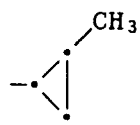 | Smp. 87-90°C |
| 2.4 | 4-Cl | H | 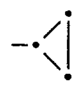 | |
| 2.5 | H | H | 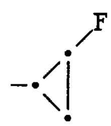 | Smp. 128-132°C |
| 2.6 | 3-F | H | 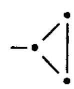 | |
| 2.7 | 4-F | H | 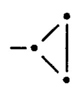 | |

2. Formulierungsbeispiele für flüssige Wirkstoffe der Formel I % = Gewichtsprozent)

| 2.1. Emulsionskonzentrate | | | |
|---|---|---|---|
| | a) | b) | c) |
| Wirkstoff aus der Tabelle 1 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | | | | |
|---|---|---|---|---|
| | a) | b) | c) | d) |
| Wirkstoff aus der Tabelle 1 | 80 % | 10 % | 5 % | 95 % |
| Ethylenglykol-monomethyl-ether | 20 % | – | – | – |
| Polyethylenglykol MG 400 | – | 70 % | – | – |
| N-Methyl-2-pyrrolidon | – | 20 % | – | – |
| Epoxydiertes Kokosnussöl | – | – | 1 % | 5 % |
| Benzin (Siedegrenzen 160-190°C) | – | – | 94 % | – |
| (MG = Molekulargewicht) | | | | |

Die Lösungen sind zur Anwendung in Form kleinster Tropfen geeignet.

| 2.3. Granulate | | |
|---|---|---|
| | a) | b) |
| Wirkstoff aus der Tabelle 1 | 5 % | 10 % |
| Kaolin | 94 % | – |
| Hochdisperse Kieselsäure | 1 % | – |
| Attapulgit | – | 90 % |

Der Wirkstoff wird in Methylenchlorid gelöst, auf den Träger aufgesprüht und das Lösungsmittel anschliessend im Vakuum abgedampft.

| 2.4. Stäubemittel | | |
|---|---|---|
| | a) | b) |
| Wirkstoff aus der Tabelle 1 | 2 % | 5 % |
| Hochdisperse Kieselsäure | 1 % | 5 % |
| Talkum | 97 % | – |
| Kaolin | – | 90 % |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| 2.5. Spritzpulver | | | |
|---|---|---|---|
| | a) | b) | c) |
| Wirkstoff aus der Tabelle 1 | 25 % | 50 % | 75 % |
| Na-Ligninsulfonat | 5 % | 5 % | – |
| Na-Laurylsulfat | 3 % | – | 5 % |
| Na-Diisobutylnaphthalinsulfonat | – | 6 % | 10 % |
| Octylphenolpolyethylenglykolether (7-8 Mol Ethylenoxid) | – | 2 % | – |
| Hochdisperse Kieselsäure | 5 % | 10 % | 10 % |
| Kaolin | 62 % | 27 % | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6. Emulsions – Konzentrat | |
|---|---|
| Wirkstoff aus der Tabelle 1 | 10 % |
| Octylphenolpolyethylenglykolether (4 – 5 Mol Ethylenoxid) | 3 % |
| Ca – Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.7. Stäubemittel | | |
|---|---|---|
| | a) | b) |
| Wirkstoff aus der Tabelle 1 | 5 % | 8 % |
| Talkum | 95 % | – |
| Kaolin | – | 92 % |

Man erhält anwendungsfertige Stäubemittel, indem der Wirkstoff mit dem Träger vermischt auf einer geeigneten Mühle vermahlen wird.

| 2.8. Extruder Granulat | |
|---|---|
| Wirkstoff aus der Tabelle 1 | 10 % |
| N – Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9. Umhüllungs – Granulat | |
|---|---|
| Wirkstoff aus der Tabelle 1 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs – Granulate.

| 2.10. Suspensions – Konzentrat | |
|---|---|
| Wirkstoff aus der Tabelle 1 | 40 % |
| Ethylenglykol | 10 % |
| Nonylphenolpolyethylenglykolether (15 Mol Ethylenoxid) | 6 % |
| N – Ligninsulfonat | 10 % |
| Carboxymethylcellulose | 1 % |
| 37%ige wässrige Formaldehyd – Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions – Konzentrat, aus welchem durch Verdünnen mit Wasser Suspensionen jeder gewünschten

Konzentration hergestellt werden können.

3. Biologische Beispiele

Beispiel 3.1: Wirkung gegen Venturia inaequalis auf Apfeltrieben Residual-protektive Wirkung

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Verbindungen aus Tabelle 1 zeigen gegen Venturia gute Wirksamkeit (Befall: weniger als 20 %). So reduzierten z.B. die Verbindungen Nr. 1.1, 1.6, 1.13, 1.14, 1.59, 1.66, 1.69, 1.84, 1.87, 1.94, 1.108, 1.126, 1.145, 1.158, 1.180, 1.200 und 1.236 den Venturia-Befall auf 0 bis 10 %. Unbehandelte aber infizierte Kontrollpflanzen weisen dagegen einen Venturia-Befall von 100 % auf.

Beispiel 3.2: Wirkung gegen Botrytis cinerea an Apfelfrüchten Residual-protektive Wirkung

Künstlich verletzte Aepfel werden behandelt, indem eine aus Spritzpulver der Wirksubstanz hergestellte Spritzbrühe (0,002 % Aktivsubstanz) auf die Verletzungsstellen aufgepfropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Verbindungen aus Tabelle 1 zeigen gegen Botrytis gute Wirksamkeit (Befall: weniger als 20 %). So reduzierten z.B. die Verbindungen Nr. 1.1, 1.6, 1.13, 1.14, 1.31, 1.33, 1.35, 1.48, 1.59, 1.66, 1.69, 1.84, 1.87, 1.94, 1.108, 1.126, 1.131, 1.145, 1.158, 1.180 und 1.236 den Botrytis-Befall auf 0 bis 10%. Unbehandelte aber infizierte Kontrollpflanzen weisen dagegen einen Botrytis-Befall von 100 % auf.

Beispiel 3.3: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus Tabelle 1 zeigen gegen Erysiphae gute Wirksamkeit (Befall: weniger als 20 %). So reduzierten z.B. die Verbindungen Nr. 1.1, 1.6, 1.13, 1.14, 1.35, 1.48, 1.59, 1.66, 1.69, 1.84, 1.87, 1.94, 1.108, 1.131, 1.158 und 1.236 den Erysiphae-Befall auf 0 bis 10 %. Unbehandelte aber infizierte Kontrollpflanzen weisen dagegen einen Erysiphae-Befall von 100 % auf.

Beispiel 3.4: Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Die Verbindungen der Tabelle verhindern den Pilzbefall weitgehend (0 bis 10 % Pilzbefall).

Beispiel 3.5 Wirkung gegen Colletotrichum lagenarium auf Gurken

Gurkenpflanzen werden nach 2-wöchiger Anzucht mit einer aus Spritzpulver des Wirkstoffs hergestellten Spritzbrühe (Konzentration 0,002 %) besprüht. Nach 2 Tagen werden die Pflanzen mit einer Sporensuspension ($1,5x10^5$ Sporen/ml) des Pilzes infiziert und für 36 Stunden bei 23°C und hoher Luftfeuchtigkeit inkubiert. Die Inkubation wird dann bei normaler Luftfeuchtigkeit und ca. 22-23°C weitergeführt. Der eingetretene Pilzbefall wird 8 Tage nach der Infektion beurteilt. Unbehandelte aber infizierte Kontrollpflanzen weisen einen Pilzbefall von 100 % auf.

Verbindungen aus Tabelle 1 zeigen gute Wirksamkeit und verhindern die Ausbreitung des Krankheits-befalls. Der Pilzbefall wird auf 20 % oder weniger zurückgedrängt.

Beispiel 3.6. a) Kontakt-Wirkung gegen Nephotettix cincticeps und Nilaparvate lugens (Nymphen);

Der Test wird an wachsenden Reis-Pflanzen durchgeführt. Dazu werden jeweils 4 Pflanzen (14-20 Tage alt) mit einer Höhe von ca. 15 cm in Töpfe (Durchmesser 5,5 cm) eingepflanzt.

Die Pflanzen werden auf einem Drehteller mit 100 ml einer wässrigen Emulsions-Zubereitung enthal-tend 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des Adultstadiums über 6 Tage an der behandelten Pflanze gehalten. Die Auswertung auf prozentuale Mortalität erfolgt 6 Tage nach der Besiedlung. Der Versuch wird bei etwa 27°C, 60 % rel. Luftfeuchtigkeit und einer Beleuchtungsperiode von 16 Stunden durchgeführt.

b) Systemische Wirkung gegen Nilaparvata lugens (Wasser)

Etwa 10 Tage alte Reispflanzen (ca. 10 cm hoch) werden in einen Plastik-Becher eingestellt, der 150 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reis-pflanze wird durch ein Loch in dem Plastikdeckel in die wässsrige Test-Zubereitung geschoben. Dann wird die Reispflanze mit 20 Nymphen von Nilaparvata lugens im N 2 bis N 3 Stadium besiedelt und mit einem Plastikzylinder abgedeckt. Der Versuch wird bei ca. 26°C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff die Testtiere an den oberen Pflanzenteilen abtötet.

Verbindungen aus Tabelle 1 zeigen sowohl im Test a) wie b) eine stark abtötende Wirkung auf die Reisschädlinge. Die Mortalitätsrate liegt bei 80 % oder mehr. Mit den Verbindungen Nr. 1.1, 1.6, 1.14, 1.59, 1.66, 1.87, 1.94, 1.108 und 1.236 wurde fast vollständige Abtötung (98-100 %) erzielt.

**Patentansprüche**

**Patentansprüche für folgende Vertragsstaaten : AT, BE, CH, DE, FR, GB, GR, IT, LI, LU, NL, SE**

**1.** Eine Verbindung der Formel I unter Einschluss ihrer Säureadditionssalze und Metallsalzkomplexe

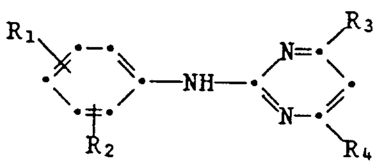

(I)

in welcher bedeuten:

$R_1$ Wasserstoff, Halogen, $C_1-C_3$-Alkyl, Trifluormethyl, $C_1-C_3$-Alkoxy oder $C_1-C_3$ Halogenalkoxy;

$R_2$ Wasserstoff, Halogen, $C_1-C_3$-Alkyl, Trifluormethyl oder $C_1-C_3$-Alkoxy;

$R_3$ Wasserstoff, $C_1-C_4$-Alkyl oder durch Halogen oder Hydroxy substituiertes $C_1-C_4$-Alkyl, Cyclo-propyl oder durch Methyl oder Halogen bis zu dreifach gleich oder verschieden substituiertes Cyclo-propyl;

$R_4$ $C_3-C_6$-Cycloalkyl oder durch Methyl und/oder Halogen bis zu dreifach gleich oder verschieden substituiertes $C_3-C_6$-Cycloalkyl.

**2.** Eine Verbindung der Formel I gemäss Anspruch 1, worin $R_3$ und $R_4$ die genannte Bedeutung haben und $R_1$ und $R_2$ Wasserstoff darstellen.

**3.** Eine Verbindung der Formel I gemäss Anspruch 1, in welcher bedeuten:

$R_1$ Wasserstoff, Halogen, $C_1-C_3$-Alkyl, Trifluormethyl, $C_1-C_3$-Alkoxy oder $C_1-C_3$ Halogenalkoxy;

$R_2$ Wasserstoff, Halogen, $C_1-C_3$-Alkyl, Trifluormethyl oder $C_1-C_3$-Alkoxy;

$R_3$ Wasserstoff, $C_1-C_4$-Alkyl oder durch Halogen substituiertes $C_1-C_4$-Alkyl;
$R_4$ $C_3-C_6$-Cycloalkyl oder durch Methyl oder Halogen substituiertes $C_3-C_6$-Cycloalkyl.

**4.** Eine Verbindung der Formel I gemäss Anspruch 3, in welcher bedeuten:
$R_1$ Wasserstoff, Fluor, Chlor, Brom, Methyl, Ethyl, Trifluormethyl, Methoxy, Ethoxy oder Halogenmethoxy;
$R_2$ Wasserstoff, Fluor, Chlor, Methyl, Trifluormethyl, Methoxy oder Ethoxy;
$R_3$ Wasserstoff, Methyl, Ethyl, n-Propyl oder sek.-Butyl; oder durch Fluor, Chlor oder Brom substituiertes Methyl;
$R_4$ $C_3-C_6$-Cycloalkyl oder durch Methyl, Fluor, Chlor oder Brom substituiertes $C_3-C_6$-Cycloalkyl.

**5.** Eine Verbindung der Formel I nach Anspruch 1, in welcher bedeuten:
$R_1$ Wasserstoff, Fluor, Chlor, Methyl, Trifluormethyl, Methoxy oder Difluormethoxy;
$R_2$ Wasserstoff, Fluor, Chlor, Methyl, Trifluormethyl oder Methoxy;
$R_3$ Wasserstoff, $C_1-C_3$-Alkyl, durch Halogen oder Hydroxy substituiertes $C_1-C_2$-Alkyl, Cyclopropyl oder durch Methyl oder Halogen substituiertes Cyclopropyl;
$R_4$ $C_3-C_6$-Cycloalkyl oder durch Methyl und/oder Halogen bis zu dreifach gleich oder verschieden substituiertes $C_3-C_4$-Cycloalkyl.

**6.** Eine Verbindung der Formel I nach Anspruch 1, in welcher bedeuten:
$R_1$ und $R_2$ Wasserstoff;
$R_3$ $C_1-C_3$-Alkyl, durch Fluor, Chlor, Brom oder Hydroxy substituiertes Methyl, Cyclopropyl oder durch Methyl, Fluor, Chlor oder Brom substituiertes Cyclopropyl;
$R_4$ $C_3-C_4$-Cycloalkyl oder durch Methyl und/oder Fluor, Chlor, Brom bis zu dreifach gleich oder verschieden substituiertes $C_3-C_4$-Cycloalkyl.

**7.** Eine Verbindung gemäss Anspruch 3, ausgewählt aus
2-Phenylamino-4-methyl-6-cyclopropyl-pyrimidin,
2-Phenylamino-4-ethyl-6-cyclopropyl-pyrimidin,
2-Phenylamino-4-methyl-6-(2-methylcyclopropyl)-pyrimidin und
2-(p-Fluorphenylamino)-4-methyl-6-cyclopropyl-pyrimidin.

**8.** Eine Verbindung gemäss Anspruch 1, ausgewählt aus
2-Phenylamino-4,6-bis(cyclopropyl)-pyrimidin,
2-Phenylamino-4-hydroxymethyl-6-cyclopropyl-pyrimidin,
2-Phenylamino-4-fluormethyl-6-cyclopropyl-pyrimidin,
2-Phenylamino-4-hydroxymethyl-6-(2-methylcyclopropyl)-pyrimidin,
2-Phenylamino-4-methyl-6-(2-fluorcyclopropyl)-pyrimidin,
2-Phenylamino-4-methyl-6-(2-chlorcyclopropyl)-pyrimidin,
2-Phenylamino-4-methyl-6-(2-difluorcyclopropyl)-pyrimidin,
2-Phenylamino-4-fluormethyl-6-(2-fluorcyclopropyl)-pyrimidin,
2-Phenylamino-4-fluormethyl-6-(2-chlorcyclopropyl)-pyrimidin,
2-Phenylamino-4-fluormethyl-6-(2-methylcyclopropyl)-pyrimidin und
2-Phenylamino-4-ethyl-6-(2-methylcyclopropyl)-pyrimidin.

**9.** Verfahren zur Herstellung einer Verbindung der Formel I, dadurch gekennzeichnet, dass man
1) ein Phenylguanidinsalz der Formel IIa

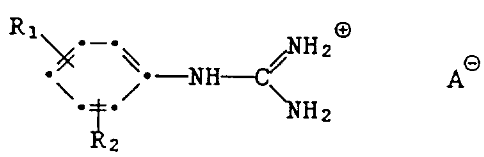

(IIa)

oder das zugrundeliegende Guanidin der Formel IIb

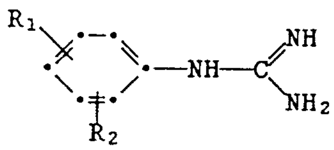

(IIb)

mit einem Diketon der Formel III

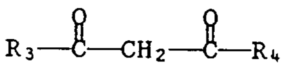

(III)

mit oder ohne Lösungsmittel bei Temperaturen von 60 °C bis 160 °C reagieren lässt, oder
2) Harnstoff mit einem Diketon der Formel III in Gegenwart einer Säure in einem inerten Lösungsmittel bei Temperaturen von 20 °C bis 140 °C zur Reaktion bringt und danach bei Rückflusstemperatur zu einer Pyrimidinverbindung der Formel V

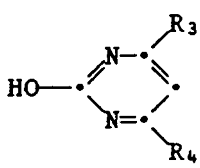

(V)

cyclisiert, die OH-Gruppe in der erhaltenen Verbindung mit überschüssigem $POHal_3$ in Gegenwart oder Abwesenheit eines Lösungsmittels bei Temperaturen von 50 °C bis 110 °C gegen Halogen austauscht

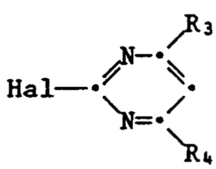

(VI)

wobei Hal in vorstehenden Formeln Halogen bedeutet, und die erhaltene Verbindung der Formel VI weiter mit einer Anilinverbindung der Formel VII

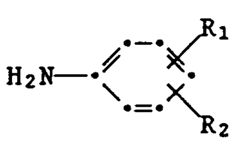

(VII)

bei 60 °C bis 120 °C je nach Verfahrensbedingungen entweder
a) in Gegenwart eines Protonenakzeptors mit oder ohne Lösungsmittel oder b) in Gegenwart einer Säure in einem inerten Lösungsmittel umsetzt; oder

3) ein Guanidinsalz der Formel VIII

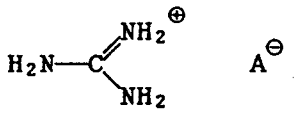

(VIII)

mit einem Diketon der Formel III

a) ohne Lösungsmittel bei Temperaturen von 100 ° C bis 160 ° C, oder

b) in einem inerten Lösungsmittel bei Temperaturen von 30 ° C bis 140 ° C zu einer Pyrimidinverbindung der Formel IX

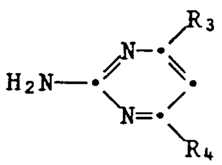

(IX)

cyclisiert, und die erhaltene Verbindung mit einer Verbindung der Formel X

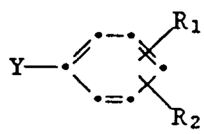

(X)

unter Abspaltung von HY in Gegenwart eines Protonenakzeptors in aprotischen Lösungsmitteln bei Temperaturen von 30 ° C bis 140 ° C umsetzt, wobei in den Formeln II bis X die Substituenten $R_1$ bis $R_4$ die unter Formel I angegebenen Bedeutungen besitzen, $A^{\ominus}$ ein Säureanion und Y Halogen darstellen.

**10.** Mittel zur Bekämpfung oder Verhütung eines Befalls durch schädliche Insekten oder Mikroorganismen, dadurch gekennzeichnet, dass es als aktive Komponente mindestens eine Verbindung der Formel I gemäss Anspruch 1 enthält, zusammen mit einem geeigneten Trägermaterial.

**11.** Mittel gemäss Anspruch 10, dadurch gekennzeichnet, dass es als aktive Komponente mindestens eine Verbindung der Formel I gemäss Anspruch 2 enthält.

**12.** Mittel gemäss Anspruch 10, dadurch gekennzeichnet, dass es als aktive Komponente mindestens eine Verbindung der Formel I gemäss Anspruch 3 enthält.

**13.** Mittel gemäss Anspruch 10, dadurch gekennzeichnet, dass es als aktive Komponente mindestens eine Verbindung der Formel I gemäss Anspruch 4 enthält.

**14.** Mittel gemäss Anspruch 10, dadurch gekennzeichnet, dass es als aktive Komponente mindestens eine Verbindung der Formel I gemäss einem der Ansprüche 5 bis 8 enthält.

**15.** Verfahren zur Bekämpfung oder Verhütung eines Befalls von Kulturpflanzen durch Schadinsekten oder phytopathogene Mikroorganismen, dadurch gekennzeichnet, dass man als Wirkstoff eine Verbindung der Formel I gemäss Anspruch 1 auf die Pflanze, auf Pflanzenteile oder ihren Standort appliziert.

**16.** Verfahren gemäss Anspruch 15, dadurch gekennzeichnet, dass man als Wirkstoff eine Verbindung gemäss einem der Ansprüche 2 bis 8 appliziert.

**17.** Verfahren gemäss Anspruch 15, dadurch gekennzeichnet, dass phytopathogene Pilze bekämpft werden.

**18.** Verfahren zur Herstellung eines agrochemischen Mittels gemäss Anspruch 10, dadurch gekennzeichnet, dass man mindestens eine Verbindung der Formel I gemäss Anspruch 1 mit geeigneten festen oder flüssigen Zusatzstoffen und/oder Tensiden innig vermischt.

**19.** Eine Verbindung der Formel XXI

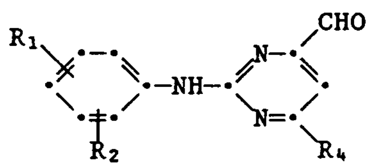

(XXI)

worin bedeuten:
$R_1$ Wasserstoff, Halogen, $C_1-C_3$-Alkyl, Trifluormethyl, $C_1-C_3$-Alkoxy oder $C_1-C_3$ Halogenalkoxy;
$R_2$ Wasserstoff, Halogen, $C_1-C_3$-Alkyl, Trifluormethyl oder $C_1-C_3$-Alkoxy;
$R_4$ $C_3-C_6$-Cycloalkyl oder durch Methyl und/oder Halogen bis zu dreifach gleich oder verschieden substituiertes $C_3-C_6$-Cycloalkyl.

**Patentansprüche für folgenden Vertragsstaat : ES**

**1.** Verfahren zur Herstellung einer Verbindung der Formel I

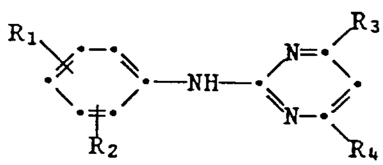

(I)

in welcher bedeuten:
$R_1$ Wasserstoff, Halogen, $C_1-C_3$-Alkyl, Trifluormethyl, $C_1-C_3$-Alkoxy oder $C_1-C_3$ Halogenalkoxy;
$R_2$ Wasserstoff, Halogen, $C_1-C_3$-Alkyl, Trifluormethyl oder $C_1-C_3$-Alkoxy;
$R_3$ Wasserstoff, $C_1-C_4$-Alkyl oder durch Halogen oder Hydroxy substituiertes $C_1-C_4$-Alkyl, Cyclopropyl oder durch Methyl oder Halogen bis zu dreifach gleich oder verschieden substituiertes Cyclopropyl;
$R_4$ $C_3-C_6$-Cycloalkyl oder durch Methyl und/oder Halogen bis zu dreifach gleich oder verschieden substituiertes $C_3-C_6$-Cycloalkyl, dadurch gekennzeichnet, dass man

1) ein Phenylguanidinsalz der Formel IIa

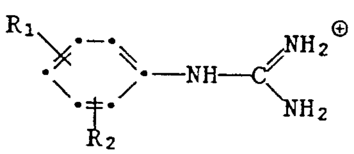

(IIa)

oder das zugrundeliegende Guanidin der Formel IIb

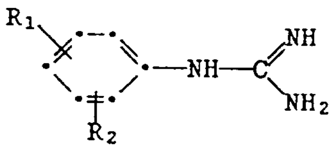

(IIb)

mit einem Diketon der Formel III

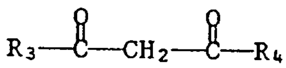

(III)

mit oder ohne Lösungsmittel bei Temperaturen von 60 ° C bis 160 ° C reagieren lässt, oder
2) Harnstoff mit einem Diketon der Formel III in Gegenwart einer Säure in einem inerten Lösungsmittel bei Temperaturen von 20 ° C bis 140 ° C zur Reaktion bringt und danach bei Rückflusstemperatur zu einer Pyrimidinverbindung der Formel V

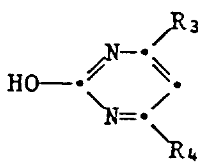

(V)

cyclisiert, die OH-Gruppe in der erhaltenen Verbindung mit überschüssigem $POHal_3$ in Gegenwart oder Abwesenheit eines Lösungsmittels bei Temperaturen von 50 ° C bis 110 ° C gegen Halogen austauscht

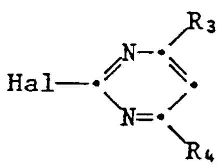

(VI)

wobei Hal in vorstehenden Formeln Halogen bedeutet, und die erhaltene Verbindung der Formel VI weiter mit einer Anilinverbindung der Formel VII

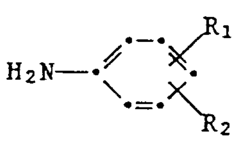

(VII)

bei 60 ° C bis 120 ° C je nach Verfahrensbedingungen entweder
a) in Gegenwart eines Protonenakzeptors mit oder ohne Lösungsmittel oder b) in Gegenwart einer Säure in einem inerten Lösungsmittel umsetzt; oder
3) ein Guanidinsalz der Formel VIII

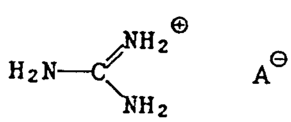

(VIII)

mit einem Diketon der Formel III

a) ohne Lösungsmittel bei Temperaturen von 100 °C bis 160 °C, oder

b) in einem inerten Lösungsmittel bei Temperaturen von 30 °C bis 140 °C zu einer Pyrimidinverbindung der Formel IX

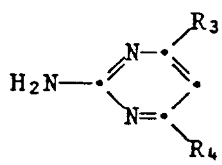

(IX)

cyclisiert, und die erhaltene Verbindung mit einer Verbindung der Formel X

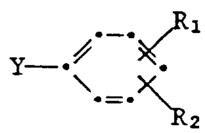

(X)

unter Abspaltung von HY in Gegenwart eines Protonenakzeptors in aprotischen Lösungsmitteln bei Temperaturen von 30 °C bis 140 °C umsetzt, wobei in den Formeln II bis X die Substituenten $R_1$ bis $R_4$ die unter Formel I angegebenen Bedeutungen besitzen, $A^{\ominus}$ ein Säureanion und Y Halogen darstellen.

**2.** Verfahren gemäss Anspruch 1, dadurch gekennzeichnet, dass Verbindungen hergestellt werden, worin $R_1$ und $R_2$ Wasserstoff darstellen, und die übrigen Substituenten die genannte Bedeutung haben.

**3.** Verfahren gemäss Anspruch 1, dadurch gekennzeichnet, dass Verbindungen hergestellt werden, worin bedeuten:
$R_1$ Wasserstoff, Halogen, $C_1-C_3$-Alkyl, Trifluormethyl, $C_1-C_3$-Alkoxy oder $C_1-C_3$ Halogenalkoxy;
$R_2$ Wasserstoff, Halogen, $C_1-C_3$-Alkyl, Trifluormethyl oder $C_1-C_3$-Alkoxy;
$R_3$ Wasserstoff, $C_1-C_4$-Alkyl oder durch Halogen substituiertes $C_1-C_4$-Alkyl;
$R_4$ $C_3-C_6$-Cycloalkyl oder durch Methyl oder Halogen substituiertes $C_3-C_6$-Cycloalkyl.

**4.** Verfahren gemäss Anspruch 1, dadurch gekennzeichnet, dass Verbindungen hergestellt werden, worin bedeuten:
$R_1$ Wasserstoff, Fluor, Chlor, Brom, Methyl, Ethyl, Trifluormethyl, Methoxy, Ethoxy oder Halogenmethoxy;
$R_2$ Wasserstoff, Fluor, Chlor, Methyl, Trifluormethyl, Methoxy oder Ethoxy;
$R_3$ Wasserstoff, Methyl, Ethyl, n-Propyl oder sek.-Butyl; oder durch Fluor, Chlor oder Brom substituiertes Methyl;
$R_4$ $C_3-C_6$-Cycloalkyl oder durch Methyl, Fluor, Chlor oder Brom substituiertes $C_3-C_6$-Cycloalkyl.

**5.** Verfahren gemäss Anspruch 1, dadurch gekennzeichnet, dass Verbindungen hergestellt werden, worin bedeuten:
$R_1$ Wasserstoff, Fluor, Chlor, Methyl, Trifluormethyl, Methoxy oder Difluormethoxy;
$R_2$ Wasserstoff, Fluor, Chlor, Methyl, Trifluormethyl oder Methoxy;
$R_3$ Wasserstoff, $C_1-C_3$-Alkyl, durch Halogen oder Hydroxy substituiertes $C_1-C_2$-Alkyl, Cyclopropyl oder durch Methyl oder Halogen substituiertes Cyclopropyl;
$R_4$ $C_3-C_6$-Cycloalkyl oder durch Methyl und/oder Halogen bis zu dreifach gleich oder verschieden substituiertes $C_3-C_4$-Cycloalkyl.

**6.** Verfahren gemäss Anspruch 1, dadurch gekennzeichnet, dass Verbindungen hergestellt werden, worin bedeuten:
$R_1$ und $R_2$ Wasserstoff;
$R_3$ $C_1-C_3$-Alkyl, durch Fluor, Chlor, Brom oder Hydroxy substituiertes Methyl, Cyclopropyl oder durch Methyl, Fluor, Chlor oder Brom substituiertes Cyclopropyl;

$R_4$ $C_3-C_4$ – Cycloalkyl oder durch Methyl und/oder Fluor, Chlor, Brom bis zu dreifach gleich oder verschieden substituiertes $C_3-C_4$ – Cycloalkyl.

**7.** Verfahren gemäss Anspruch 1, dadurch gekennzeichnet, dass eine Verbindung hergestellt wird, ausgewählt aus
2 – Phenylamino – 4 – methyl – 6 – cyclopropyl – pyrimidin,
2 – Phenylamino – 4 – ethyl – 6 – cyclopropyl – pyrimidin,
2 – Phenylamino – 4 – methyl – 6 – (2 – methylcyclopropyl) – pyrimidin und
2 – (p – Fluorphenylamino) – 4 – methyl – 6 – cyclopropyl – pyrimidin.

**8.** Verfahren gemäss Anspruch 1, dadurch gekennzeichnet, dass eine Verbindung hergestellt wird, ausgewählt aus
2 – Phenylamino – 4,6 – bis(cyclopropyl) – pyrimidin,
2 – Phenylamino – 4 – hydroxymethyl – 6 – cyclopropyl – pyrimidin,
2 – Phenylamino – 4 – fluormethyl – 6 – cyclopropyl – pyrimidin,
2 – Phenylamino – 4 – hydroxymethyl – 6 – (2 – methylcyclopropyl) – pyrimidin,
2 – Phenylamino – 4 – methyl – 6 – (2 – fluorcyclopropyl) – pyrimidin,
2 – Phenylamino – 4 – methyl – 6 – (2 – chlorcyclopropyl) – pyrimidin,
2 – Phenylamino – 4 – methyl – 6 – (2 – difluorcyclopropyl) – pyrimidin,
2 – Phenylamino – 4 – fluormethyl – 6 – (2 – fluorcyclopropyl) – pyrimidin,
2 – Phenylamino – 4 – fluormethyl – 6 – (2 – chlorcyclopropyl) – pyrimidin,
2 – Phenylamno – 4 – fluormethyl – 6 – (2 – methylcyclopropyl) – pyrimidin und
2 – Phenylamino – 4 – ethyl – 6 – (2 – methylcyclopropyl) – pyrimidin.

**9.** Mittel zur Bekämpfung oder Verhütung eines Befalls durch schädliche Insekten oder Mikroorganismen, dadurch gekennzeichnet, dass es, zusammen mit einem geeigneten Trägermaterial, als aktive Komponente mindestens eine Verbindung der Formel I enthält

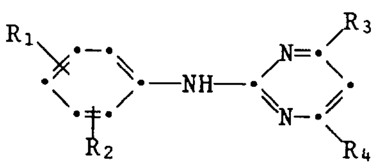

(I)

in welcher bedeuten:
$R_1$ Wasserstoff, Halogen, $C_1-C_3$ – Alkyl, Trifluormethyl, $C_1-C_3$ – Alkoxy oder $C_1-C_3$ Halogenalkoxy;
$R_2$ Wasserstoff, Halogen, $C_1-C_3$ – Alkyl, Trifluormethyl oder $C_1-C_3$ – Alkoxy;
$R_3$ Wasserstoff, $C_1-C_4$ – Alkyl oder durch Halogen oder Hydroxy substituiertes $C_1-C_4$ – Alkyl, Cyclopropyl oder durch Methyl oder Halogen bis zu dreifach gleich oder verschieden substituiertes Cyclopropyl;
$R_4$ $C_3-C_6$ – Cycloalkyl oder durch Methyl und/oder Halogen bis zu dreifach gleich oder verschieden substituiertes $C_3-C_6$ – Cycloalkyl.

**10.** Mittel gemäss Anspruch 9, dadurch gekennzeichnet, dass es als aktive Komponente mindestens eine Verbindung der Formel I enthält, worin $R_3$ und $R_4$ die genannte Bedeutung haben und $R_1$ und $R_2$ Wasserstoff darstellen.

**11.** Mittel gemäss Anspruch 9, dadurch gekennzeichnet, dass es als aktive Komponente mindestens eine Verbindung der Formel I enthält, in welcher bedeuten:
$R_1$ Wasserstoff, Halogen, $C_1-C_3$ – Alkyl, Trifluormethyl, $C_1-C_3$ – Alkoxy oder $C_1-C_3$ Halogenalkoxy;
$R_2$ Wasserstoff, Halogen, $C_1-C_3$ – Alkyl, Trifluormethyl oder $C_1-C_3$ – Alkoxy;
$R_3$ Wasserstoff, $C_1-C_4$ – Alkyl oder durch Halogen substituiertes $C_1-C_4$ – Alkyl,
$R_4$ $C_3-C_6$ – Cycloalkyl oder durch Methyl oder Halogen substituiertes $C_3-C_6$ – Cycloalkyl.

**12.** Mittel gemäss Anspruch 9, dadurch gekennzeichnet, dass es als aktive Komponente mindestens eine Verbindung der Formel I enthält, in welcher bedeuten:

$R_1$ Wasserstoff, Fluor, Chlor, Brom, Methyl, Ethyl, Trifluormethyl, Methoxy, Ethoxy oder Halogenmethoxy;
$R_2$ Wasserstoff, Fluor, Chlor, Methyl, Trifluormethyl, Methoxy oder Ethoxy;
$R_3$ Wasserstoff, Methyl, Ethyl, n-Propyl oder sek.-Butyl; oder durch Fluor, Chlor oder Brom substituiertes Methyl;
$R_4$ $C_3-C_6$-Cycloalkyl oder durch Methyl, Fluor, Chlor oder Brom substituiertes $C_3-C_6$-Cycloalkyl.

**13.** Mittel gemäss Anspruch 9, dadurch gekennzeichnet, dass es als aktive Komponente mindestens eine Verbindung der Formel I enthält, in welcher bedeuten:
$R_1$ Wasserstoff, Fluor, Chlor, Methyl, Trifluormethyl, Methoxy oder Difluormethoxy;
$R_2$ Wasserstoff, Fluor, Chlor, Methyl, Trifluormethyl oder Methoxy;
$R_3$ Wasserstoff, $C_1-C_3$-Alkyl, durch Halogen oder Hydroxy substituiertes $C_1-C_2$-Alkyl, Cyclopropyl oder durch Methyl oder Halogen substituiertes Cyclopropyl;
$R_4$ $C_3-C_6$-Cycloalkyl oder durch Methyl und/oder Halogen bis zu dreifach gleich oder verschieden substituiertes $C_3-C_4$-Cycloalkyl.

**14.** Mittel gemäss Anspruch 9, dadurch gekennzeichnet, dass es als aktive Komponente mindestens eine Verbindung der Formel I enthält, in welcher bedeuten:
$R_1$ und $R_2$ Wasserstoff;
$R_3$ $C_1-C_3$-Alkyl, durch Fluor, Chlor, Brom oder Hydroxy substituiertes Methyl, Cyclopropyl oder durch Methyl, Fluor, Chlor oder Brom substituiertes Cyclopropyl;
$R_4$ $C_3-C_4$-Cycloalkyl oder durch Methyl und/oder Fluor, Chlor, Brom bis zu dreifach gleich oder verschieden substituiertes $C_3-C_4$-Cycloalkyl.

**15.** Mittel gemäss Anspruch 9, dadurch gekennzeichnet, dass es als aktive Komponente mindestens eine Verbindung der Formel I enthält, ausgewählt aus
2-Phenylamino-4-methyl-6-cyclopropyl-pyrimidin,
2-Phenylamino-4-ethyl-6-cyclopropyl-pyrimidin,
2-Phenylamino-4-methyl-6-(2-methylcyclopropyl)-pyrimidin
und
2-(p-Fluorphenylamino)-4-methyl-6-cyclopropyl-pyrimidin.

**16.** Mittel gemäss Anspruch 9, dadurch gekennzeichnet, dass es als aktive Komponente mindestens eine Verbindung der Formel I enthält, ausgewählt aus
2-Phenylamino-4,6-bis(cyclopropyl)-pyrimidin,
2-Phenylamino-4-hydroxymethyl-6-cyclopropyl-pyrimidin,
2-Phenylamino-4-fluormethyl-6-cyclopropyl-pyrimidin,
2-Phenylamino-4-hydroxymethyl-6-(2-methylcyclopropyl)-pyrimidin,
2-Phenylamino-4-methyl-6-(2-fluorcyclopropyl)-pyrimidin,
2-Phenylamino-4-methyl-6-(2-chlorcyclopropyl)-pyrimidin,
2-Phenylamino-4-methyl-6-(2-difluorcyclopropyl)-pyrimidin,
2-Phenylamino-4-fluormethyl-6-(2-fluorcyclopropyl)-pyrimidin,
2-Phenylamino-4-fluormethyl-6-(2-chlorcyclopropyl)-pyrimidin,
2-Phenylamno-4-fluormethyl-6-(2-methylcyclopropyl)-pyrimidin und
2-Phenylamino-4-ethyl-6-(2-methylcyclopropyl)-pyrimidin.

**17.** Verfahren zur Bekämpfung oder Verhütung eines Befalls von Kulturpflanzen durch Schadinsekten oder phytopathogene Mikroorganismen, dadurch gekennzeichnet, dass man als Wirkstoff eine Verbindung der Formel I gemäss Anspruch 9 auf die Pflanze, auf Pflanzenteile oder ihren Standort appliziert.

**18.** Verfahren gemäss Anspruch 17, dadurch gekennzeichnet, dass man als Wirkstoff eine Verbindung gemäss einem der Ansprüche 10 bis 16 appliziert.

**19.** Verfahren gemäss Anspruch 17, dadurch gekennzeichnet, dass phytopathogene Pilze bekämpft werden.

**20.** Verfahren zur Herstellung eines agrochemischen Mittels gemäss Anspruch 9, dadurch gekennzeichnet, dass man mindestens eine Verbindung der Formel I gemäss Anspruch 9 mit geeigneten festen oder

flüssigen Zusatzstoffen und/oder Tensiden innig vermischt.

**Claims**

**Claims for the following Contracting States : AT, BE, CH, DE, FR, GB, GR, IT, LI, LU, NL, SE**

**1.** A compound of formula I including an acid addition salt or a metal salt complex thereof

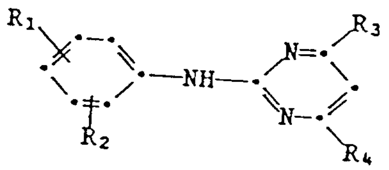

(I)

in which:
$R_1$ is hydrogen, halogen, $C_1-C_3$alkyl, trifluoromethyl, $C_1-C_3$alkoxy or $C_1-C_3$haloalkoxy;
$R_2$ is hydrogen, halogen, $C_1-C_3$alkyl, trifluoromethyl or $C_1-C_3$alkoxy;
$R_3$ is hydrogen; $C_1-C_4$alkyl; or $C_1-C_4$alkyl substituted by halogen or by hydroxy; cyclopropyl; or cyclopropyl mono- to tri-substituted by the same or different substituents selected from methyl and halogen; and
$R_4$ is $C_3-C_6$cycloalkyl or $C_3-C_6$cycloalkyl mono- to tri-substituted by the same or different substituents selected from methyl and halogen.

**2.** A compound of formula I according to claim 1, in which $R_3$ and $R_4$ have the meanings given and $R_1$ and $R_2$ are hydrogen.

**3.** A compound of formula I according to claim 1, in which:
$R_1$ is hydrogen, halogen, $C_1-C_3$alkyl, trifluoromethyl, $C_1-C_3$alkoxy or $C_1-C_3$haloalkoxy;
$R_2$ is hydrogen, halogen, $C_1-C_3$alkyl, trifluoromethyl or $C_1-C_3$alkoxy;
$R_3$ is hydrogen, $C_1-C_4$alkyl or $C_1-C_4$alkyl substituted by halogen; and
$R_4$ is $C_3-C_6$cycloalkyl or $C_3-C_6$cycloalkyl substituted by methyl or by halogen.

**4.** A compound of formula I according to claim 3, in which:
$R_1$ is hydrogen, fluorine, chlorine, bromine, methyl, ethyl, trifluoromethyl, methoxy, ethoxy or halomethoxy;
$R_2$ is hydrogen, fluorine, chlorine, methyl, trifluoromethyl, methoxy or ethoxy;
$R_3$ is hydrogen, methyl, ethyl, n-propyl or sec-butyl, or is methyl substituted by fluorine, chlorine or by bromine; and
$R_4$ is $C_3-C_6$cycloalkyl or $C_3-C_6$cycloalkyl substituted by methyl, fluorine, chlorine or by bromine.

**5.** A compound of formula I according to claim 1, in which:
$R_1$ is hydrogen, fluorine, chlorine, methyl, trifluoromethyl, methoxy or difluoromethoxy;
$R_2$ is hydrogen, fluorine, chlorine, methyl, trifluoromethyl or methoxy;
$R_3$ is hydrogen; $C_1-C_3$alkyl; $C_1-C_2$alkyl substituted by halogen or by hydroxy; cyclopropyl; or cyclopropyl substituted by methyl or by halogen; and
$R_4$ is $C_3-C_6$cycloalkyl or $C_3-C_4$cycloalkyl mono- to tri-substituted by the same or different substituents selected from methyl and halogen.

**6.** A compound of formula I according to claim 1, in which:
$R_1$ and $R_2$ are hydrogen;
$R_3$ is $C_1-C_3$alkyl; methyl substituted by fluorine, chlorine, bromine or by hydroxy; cyclopropyl; or cyclopropyl substituted by methyl, fluorine, chlorine or by bromine; and
$R_4$ is $C_3-C_4$cycloalkyl or $C_3-C_4$cycloalkyl mono- to tri-substituted by the same or different substituents selected from methyl and fluorine, chlorine and bromine.

**7.** A compound according to claim 3 selected from
2-phenylamino-4-methyl-6-cyclopropylpyrimidine,

2-phenylamino-4-ethyl-6-cyclopropylpyrimidine,
2-phenylamino-4-methyl-6-(2-methylcyclopropyl)-pyrimidine, and
2-(p-fluorophenylamino)-4-methyl-6-cyclopropylpyrimidine.

**8.** A compound according to claim 1, selected from
2-phenylamino-4,6-bis(cyclopropyl)pyrimidine,
2-phenylamino-4-hydroxymethyl-6-cyclopropylpyrimidine,
2-phenylamino-4-fluoromethyl-6-cyclopropylpyrimidine,
2-phenylamino-4-hydroxymethyl-6-(2-methylcyclopropyl)-pyrimidine,
2-phenylamino-4-methyl-6-(2-fluorocyclopropyl)-pyrimidine,
2-phenylamino-4-methyl-6-(2-chlorocyclopropyl)-pyrimidine,
2-phenylamino-4-methyl-6-(2-difluorocyclopropyl)-pyrimidine,
2-phenylamino-4-fluoromethyl-6-(2-fluorocyclopropyl)-pyrimidine,
2-phenylamino-4-fluoromethyl-6-(2-chlorocyclopropyl)-pyrimidine,
2-phenylamino-4-fluoromethyl-6-(2-methylcyclopropyl)-pyrimidine and
2-phenylamino-4-ethyl-6-(2-methylcyclopropyl)-pyrimidine.

**9.** A process for the preparation of a compound of formula I, which comprises
1) reacting a phenylguanidine salt of formula IIa

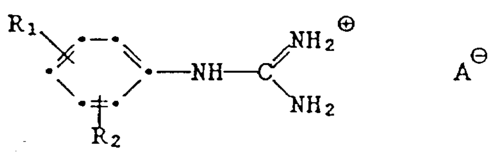

(IIa)

or the guanidine base of formula IIb

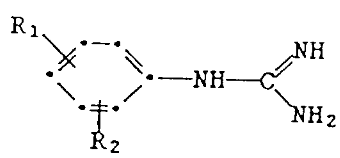

(IIb)

with a diketone of formula III

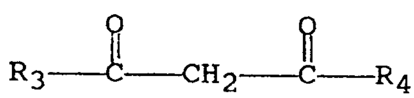

(III),

with or without solvents, at temperatures of from 60°C to 160°C; or
2) reacting urea with a diketone of formula III in the presence of an acid in an inert solvent at temperatures of from 20°C to 140°C, and then cyclising at reflux temperature to give a pyrimidine compound of formula V

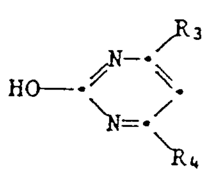

(V),

exchanging the OH group in the resulting compound for halogen using excess $POHal_3$, in the presence or in the absence of a solvent, at temperatures of from 50°C to 110°C, to yield

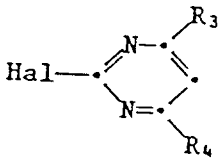

(VI),

Hal in the above formulae being halogen, and further reacting the resulting compound of formula VI with an aniline compound of formula VII

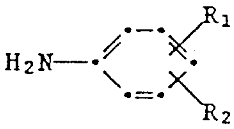

(VII)

at from 60 ° C to 120 ° C, depending on the reaction conditions either

a) in the presence of a proton acceptor, with or without solvents, or

b) in the presence of an acid in an inert solvent; or

3) cyclising a guanidine salt of formula VIII

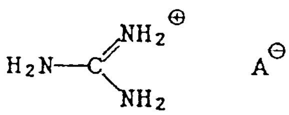

(VIII)

with a diketone of formula III

a) without solvents at temperatures of from 100 ° C to 160 ° C, or

b) in an inert solvent at temperatures of from 30 ° C to 140 ° C,

to give a pyrimidine compound of formula IX

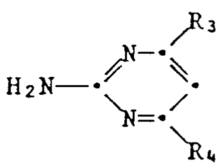

(IX)

and reacting the resulting compound with a compound of formula X

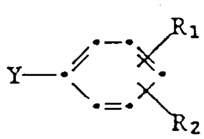

(X)

in the presence of a proton acceptor in aprotic solvents at temperatures of from 30 ° C to 140 ° C to remove HY, the substituents $R_1$ to $R_4$ in formulae II to X being as defined for formula I, $A^{\ominus}$ being an acid anion and Y being halogen.

**10.** A composition for controlling or preventing attack by noxious insects or microorganisms, which comprises as active ingredient at least one compound of formula I according to claim 1, together with a suitable carrier.

**11.** A composition according to claim 10, which comprises as active ingredient at least one compound of formula I according to claim 2.

**12.** A composition according to claim 10, which comprises as active ingredient at least one compound of formula I according to claim 3.

**13.** A composition according to claim 10, which comprises as active ingredient at least one compound of formula I according to claim 4.

**14.** A composition according to claim 10, which comprises as active ingredient at least one compound of formula I according to any one of claims 5 to 8.

**15.** A method of controlling or preventing an attack on cultivated plants by noxious insects or phytopathogenic microorganisms, which comprises applying as active ingredient to the plant, to parts of plants or to the locus thereof, a compound of formula I according to claim 1.

**16.** A method according to claim 15, which comprises applying as active ingredient a compound according to any one of claims 2 to 8.

**17.** A method according to claim 15, wherein phytopathogenic fungi are controlled.

**18.** A process for the preparation of an agrochemical composition according to claim 10, which comprises intimately mixing at least one compound of formula I according to claim 1 with suitable solid or liquid adjuvants and/or surfactants.

**19.** A compound of formula XXI

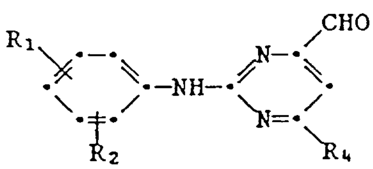

(XXI)

in which:
$R_1$ is hydrogen, halogen, $C_1-C_3$alkyl, trifluoromethyl, $C_1-C_3$alkoxy or $C_1-C_3$haloalkoxy;
$R_2$ is hydrogen, halogen, $C_1-C_3$alkyl, trifluoromethyl or $C_1-C_3$alkoxy; and
$R_4$ is $C_3-C_6$cycloalkyl or $C_3-C_6$cycloalkyl mono- to tri-substituted by the same or different substituents selected from methyl and halogen.

**Claims for the following Contracting State : ES**

**1.** A process for the preparation of a compound of formula I

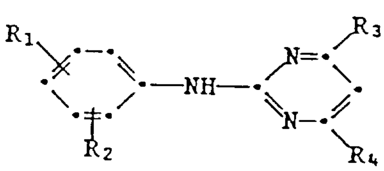

(I)

in which:
$R_1$ is hydrogen, halogen, $C_1-C_3$alkyl, trifluoromethyl, $C_1-C_3$alkoxy or $C_1-C_3$haloalkoxy;
$R_2$ is hydrogen, halogen, $C_1-C_3$alkyl, trifluoromethyl or $C_1-C_3$alkoxy;
$R_3$ is hydrogen; $C_1-C_4$alkyl; or $C_1-C_4$alkyl substituted by halogen or by hydroxy; cyclopropyl; or cyclopropyl mono- to tri-substituted by the same or different substituents selected from methyl and halogen; and
$R_4$ is $C_3-C_6$cycloalkyl or $C_3-C_6$cycloalkyl mono- to tri-substituted by the same or different substituents selected from methyl and halogen, which comprises

1) reacting a phenylguanidine salt of formula IIa

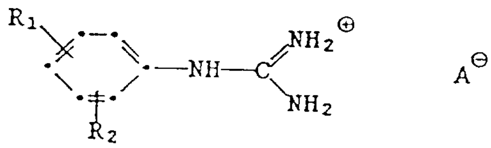

(IIa)

or the guanidine base of formula IIb

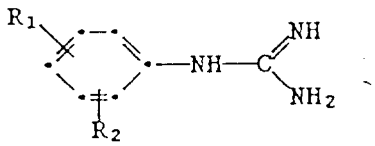

(IIb)

with a diketone of formula III

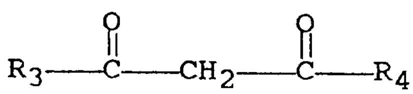

(III),

with or without solvents, at temperatures of from 60 ° C to 160 ° C; or

2) reacting urea with a diketone of formula III in the presence of an acid in an inert solvent at temperatures of from 20 ° C to 140 ° C, and then cyclising at reflux temperature to give a pyrimidine compound of formula V

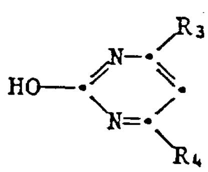

(V),

exchanging the OH group in the resulting compound for halogen using excess $POHal_3$, in the presence or in the absence of a solvent, at temperatures of from 50 ° C to 110 ° C, to yield

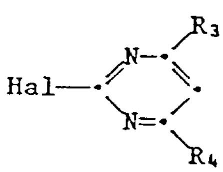

(VI),

Hal in the above formulae being halogen, and further reacting the resulting compound of formula VI with an aniline compound of formula VII

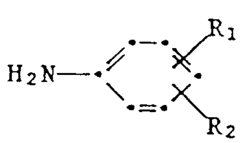

(VII)

at from 60 ° C to 120 ° C, depending on the reaction conditions either

a) in the presence of a proton acceptor, with or without solvents, or

b) in the presence of an acid in an inert solvent; or

3) cyclising a guanidine salt of formula VIII

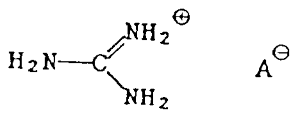

(VIII)

with a diketone of formula III

a) without solvents at temperatures of from 100 ° C to 160 ° C, or

b) in an inert solvent at temperatures of from 30 ° C to 140 ° C,

to give a pyrimidine compound of formula IX

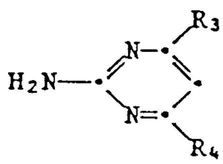

(IX)

and reacting the resulting compound with a compound of formula X

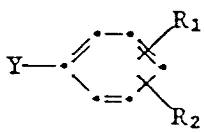

(X)

in the presence of a proton acceptor in aprotic solvents at temperatures of from 30 ° C to 140 ° C to remove HY, the substituents $R_1$ to $R_4$ in formulae II to X being as defined for formula I, $A^{\ominus}$ being an acid anion and Y being halogen.

2. A process according to claim 1, which comprises preparing a compound in which $R_1$ and $R_2$ are hydrogen and the other substituents are as defined.

3. A process according to claim 1, which comprises preparing a compound in which:
$R_1$ is hydrogen, halogen, $C_1-C_3$alkyl, trifluoromethyl, $C_1-C_3$alkoxy or $C_1-C_3$haloalkoxy;
$R_2$ is hydrogen, halogen, $C_1-C_3$alkyl, trifluoromethyl or $C_1-C_3$alkoxy;
$R_3$ is hydrogen, $C_1-C_4$alkyl or $C_1-C_4$alkyl substituted by halogen; and
$R_4$ is $C_3-C_6$cycloalkyl or $C_3-C_6$cycloalkyl substituted by methyl or by halogen.

4. A process according to claim 1, which comprises preparing a compound in which:
$R_1$ is hydrogen, fluorine, chlorine, bromine, methyl, ethyl, trifluoromethyl, methoxy, ethoxy or halomethoxy;
$R_2$ is hydrogen, fluorine, chlorine, methyl, trifluoromethyl, methoxy or ethoxy;
$R_3$ is hydrogen, methyl, ethyl, n-propyl or sec-butyl, or is methyl substituted by fluorine, chlorine or by bromine; and
$R_4$ is $C_3-C_6$cycloalkyl or $C_3-C_6$cycloalkyl substituted by methyl, fluorine, chlorine or by bromine.

5. A process according to claim 1, which comprises preparing a compound in which:
$R_1$ is hydrogen, fluorine, chlorine, methyl, trifluoromethyl, methoxy or difluoromethoxy;
$R_2$ is hydrogen, fluorine, chlorine, methyl, trifluoromethyl or methoxy;
$R_3$ is hydrogen; $C_1-C_3$alkyl; $C_1-C_2$alkyl substituted by halogen or by hydroxy; cyclopropyl; or cyclopropyl substituted by methyl or by halogen; and
$R_4$ is $C_3-C_6$cycloalkyl or $C_3-C_4$cycloalkyl mono- to tri-substituted by the same or different substituents selected from methyl and halogen.

**6.** A process according to claim 1, which comprises preparing a compound in which:
$R_1$ and $R_2$ are hydrogen;
$R_3$ is $C_1-C_3$alkyl; methyl substituted by fluorine, chlorine, bromine or by hydroxy; cyclopropyl; or cyclopropyl substituted by methyl, fluorine, chlorine or by bromine; and
$R_4$ is $C_3-C_4$cycloalkyl or $C_3-C_4$cycloalkyl mono- to tri-substituted by the same or different substituents selected from methyl and fluorine, chlorine and bromine.

**7.** A process according to claim 1, which comprises preparing a compound selected from
2-phenylamino-4-methyl-6-cyclopropylpyrimidine,
2-phenylamino-4-ethyl-6-cyclopropylpyrimidine,
2-phenylamino-4-methyl-6-(2-methylcyclopropyl)-pyrimidine, and
2-(p-fluorophenylamino)-4-methyl-6-cyclopropylpyrimidine.

**8.** A process according to claim 1, which comprises preparing a compound selected from
2-phenylamino-4,6-bis(cyclopropyl)pyrimidine,
2-phenylamino-4-hydroxymethyl-6-cyclopropylpyrimidine,
2-phenylamino-4-fluoromethyl-6-cyclopropylpyrimidine,
2-phenylamino-4-hydroxymethyl-6-(2-methylcyclopropyl)-pyrimidine,
2-phenylamino-4-methyl-6-(2-fluorocyclopropyl)-pyrimidine,
2-phenylamino-4-methyl-6-(2-chlorocyclopropyl)-pyrimidine,
2-phenylamino-4-methyl-6-(2-difluorocyclopropyl)-pyrimidine,
2-phenylamino-4-fluoromethyl-6-(2-fluorocyclopropyl)-pyrimidine,
2-phenylamino-4-fluoromethyl-6-(2-chlorocyclopropyl)-pyrimidine,
2-phenylamino-4-fluoromethyl-6-(2-methylcyclopropyl)-pyrimidine and
2-phenylamino-4-ethyl-6-(2-methylcyclopropyl)-pyrimidine.

**9.** A composition for controlling or preventing attack by noxious insects or microorganisms, which comprises as active ingredient, together with a suitable carrier, at least one compound of formula I

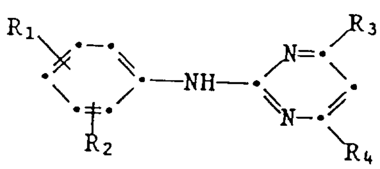

(I)

in which:
$R_1$ is hydrogen, halogen, $C_1-C_3$alkyl, trifluoromethyl, $C_1-C_3$alkoxy or $C_1-C_3$haloalkoxy;
$R_2$ is hydrogen, halogen, $C_1-C_3$alkyl, trifluoromethyl or $C_1-C_3$alkoxy;
$R_3$ is hydrogen; $C_1-C_4$alkyl; or $C_1-C_4$alkyl substituted by halogen or by hydroxy; cyclopropyl; or cyclopropyl mono- to tri-substituted by the same or different substituents selected from methyl and halogen; and
$R_4$ is $C_3-C_6$cycloalkyl or $C_3-C_6$cycloalkyl mono- to tri-substituted by the same or different substituents selected from methyl and halogen.

**10.** A composition according to claim 9, which comprises as active ingredient at least one compound of formula I in which $R_3$ and $R_4$ have the meanings given and $R_1$ and $R_2$ are hydrogen.

**11.** A composition according to claim 9, which comprises as active ingredient at least one compound of formula I in which:
$R_1$ is hydrogen, halogen, $C_1-C_3$alkyl, trifluoromethyl, $C_1-C_3$alkoxy or $C_1-C_3$haloalkoxy;
$R_2$ is hydrogen, halogen, $C_1-C_3$alkyl, trifluoromethyl or $C_1-C_3$alkoxy;
$R_3$ is hydrogen, $C_1-C_4$alkyl or $C_1-C_4$alkyl substituted by halogen; and
$R_4$ is $C_3-C_6$cycloalkyl or $C_3-C_6$cycloalkyl substituted by methyl or by halogen.

**12.** A composition according to claim 9, which comprises as active ingredient at least one compound of formula I in which:

$R_1$ is hydrogen, fluorine, chlorine, bromine, methyl, ethyl, trifluoromethyl, methoxy, ethoxy or halomethoxy;
$R_2$ is hydrogen, fluorine, chlorine, methyl, trifluoromethyl, methoxy or ethoxy;
$R_3$ is hydrogen, methyl, ethyl, n-propyl or sec-butyl, or is methyl substituted by fluorine, chlorine or by bromine; and
$R_4$ is $C_3-C_6$cycloalkyl or $C_3-C_6$cycloalkyl substituted by methyl, fluorine, chlorine or by bromine.

**13.** A composition according to claim 9, which comprises as active ingredient at least one compound of formula I in which:
$R_1$ is hydrogen, fluorine, chlorine, methyl, trifluoromethyl, methoxy or difluoromethoxy;
$R_2$ is hydrogen, fluorine, chlorine, methyl, trifluoromethyl or methoxy;
$R_3$ is hydrogen; $C_1-C_3$alkyl; $C_1-C_2$alkyl substituted by halogen or by hydroxy; cyclopropyl; or cyclopropyl substituted by methyl or by halogen; and
$R_4$ is $C_3-C_6$cycloalkyl or $C_3-C_4$cycloalkyl mono- to tri-substituted by the same or different substituents selected from methyl and halogen.

**14.** A composition according to claim 9, which comprises as active ingredient at least one compound of formula I in which:
$R_1$ and $R_2$ are hydrogen;
$R_3$ is $C_1-C_3$alkyl; methyl substituted by fluorine, chlorine, bromine or by hydroxy; cyclopropyl; or cyclopropyl substituted by methyl, fluorine, chlorine or by bromine; and
$R_4$ is $C_3-C_4$cycloalkyl or $C_3-C_4$cycloalkyl mono- to tri-substituted by the same or different substituents selected from methyl and fluorine, chlorine and bromine.

**15.** A composition according to claim 9, which comprises as active ingredient at least one compound of formula I selected from
2-phenylamino-4-methyl-6-cyclopropylpyrimidine,
2-phenylamino-4-ethyl-6-cyclopropylpyrimidine,
2-phenylamino-4-methyl-6-(2-methylcyclopropyl)-pyrimidine, and
2-(p-fluorophenylamino)-4-methyl-6-cyclopropylpyrimidine.

**16.** A composition according to claim 9, which comprises as active ingredient at least one compound of formula I selected from
2-phenylamino-4,6-bis(cyclopropyl)pyrimidine,
2-phenylamino-4-hydroxymethyl-6-cyclopropylpyrimidine,
2-phenylamino-4-fluoromethyl-6-cyclopropylpyrimidine,
2-phenylamino-4-hydroxymethyl-6-(2-methylcyclopropyl)-pyrimidine,
2-phenylamino-4-methyl-6-(2-fluorocyclopropyl)-pyrimidine,
2-phenylamino-4-methyl-6-(2-chlorocyclopropyl)-pyrimidine,
2-phenylamino-4-methyl-6-(2-difluorocyclopropyl)-pyrimidine,
2-phenylamino-4-fluoromethyl-6-(2-fluorocyclopropyl)-pyrimidine,
2-phenylamino-4-fluoromethyl-6-(2-chlorocyclopropyl)-pyrimidine,
2-phenylamino-4-fluoromethyl-6-(2-methylcyclopropyl)-pyrimidine and
2-phenylamino-4-ethyl-6-(2-methylcyclopropyl)-pyrimidine.

**17.** A method of controlling or preventing an attack on cultivated plants by noxious insects or phytopathogenic microorganisms, which comprises applying as active ingredient to the plant, to parts of plants or to the locus thereof, a compound of formula I according to claim 9.

**18.** A method according to claim 17, which comprises applying as active ingredient a compound according to any one of claims 10 to 16.

**19.** A method according to claim 17, wherein phytopathogenic fungi are controlled.

**20.** A process for the preparation of an agrochemical composition according to claim 9, which comprises intimately mixing at least one compound of formula I according to claim 9 with suitable solid or liquid adjuvants and/or surfactants.

**Revendications**

**Revendications pour les Etats contractants suivants : AT, BE, CH, DE, FR, GB, GR, IT, LI, LU, NL, SE**

**1.** Un composé de formule I, y compris ses sels formés par addition avec des acides et ses complexes de sels métalliques

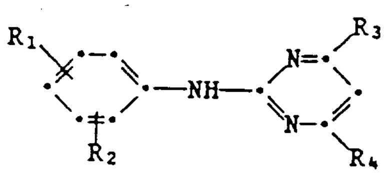

(I)

dans laquelle

$R_1$ représente l'hydrogène, un halogène, un groupe alkyle en C 1 – C 3, trifluorométhyle, alcoxy en C 1 – C 3 ou halogénoalcoxy en C 1 – C 3;

$R_2$ représente l'hydrogène, un halogène, un groupe alkyle en C 1 – C 3, trifluorométhyle ou alcoxy en C 1 – C 3;

$R_3$ représente l'hydrogène, un groupe alkyle en C 1 – C 4, ou un groupe alkyle en C 1 – C 4 substitué par un halogène ou un groupe hydroxy, un groupe cyclopropyle ou un groupe cyclopropyle portant jusqu'à trois substituants identiques ou différents choisis parmi les groupes méthyle et les halogènes;

$R_4$ représente un groupe cycloalkyle en C 3 – C 6 ou un groupe cycloalkyle en C 3 – C 6 portant jusqu'à trois substituants identiques ou différents choisis parmi les groupes méthyle et les halogènes.

**2.** Un composé de formule I de la revendication 1, dans laquelle $R_3$ et $R_4$ ont les significations indiquées ci – dessus et $R_1$ et $R_2$ représentent l'hydrogène.

**3.** Un composé de formule I de la revendication 1, dans laquelle

$R_1$ représente l'hydrogène, un halogène, un groupe alkyle en C 1 – C 3, trifluorométhyle, alcoxy en C 1 – C 3 ou halogénoalcoxy en C 1 – C 3,

$R_2$ représente l'hydrogène, un halogène, un groupe alkyle en C 1 – C 3, trifluorométhyle ou alcoxy en C 1 – C 3,

$R_3$ représente l'hydrogène, un groupe alkyle en C 1 – C 4 ou un groupe alkyle en C 1 – C 4 substitué par un halogène,

$R_4$ représente un groupe cycloalkyle en C 3 – C 6 ou un groupe cycloalkyle en C 3 – C 6 substitué par un groupe méthyle ou un halogène.

**4.** Un composé de formule I selon revendication 3, dans laquelle

$R_1$ représente l'hydrogène, le fluor, le chlore, le brome, un groupe méthyle, éthyle, trifluorométhyle, méthoxy, éthoxy ou halogénométhoxy;

$R_2$ représente l'hydrogène, le fluor, le chlore, un groupe méthyle, trifluorométhyle, méthoxy ou éthoxy;

$R_3$ représente l'hydrogène, un groupe méthyle, éthyle, n – propyle ou sec – butyle; ou un groupe méthyle substitué par le fluor, le chlore ou le brome;

$R_4$ représente un groupe cycloalkyle en C 3 – C 6 ou un groupe cycloalkyle en C 3 – C 6 substitué par un groupe méthyle, le fluor, le chlore ou le brome.

**5.** Un composé de formule I de la revendication 1, dans laquelle

$R_1$ représente l'hydrogène, le fluor, le chlore, un groupe méthyle, trifluorométhyle, méthoxy ou difluorométhoxy;

$R_2$ représente l'hydrogène, le fluor, le chlore, un groupe méthyle, trifluorométhyle ou méthoxy;

$R_3$ représente l'hydrogène, un groupe alkyle en C 1 – C 3, un groupe alkyle en C 1 – C 2 substitué par un halogène ou un groupe hydroxy, un groupe cyclopropyle ou cyclopropyle substitué par un groupe méthyle ou un halogène;

$R_4$ représente un groupe cycloalkyle en C 3 – C 6 ou un groupe cycloalkyle en C 3 – C 4 portant

jusqu'à trois substituants identiques ou différents choisis parmi les groupes méthyle et les halogènes.

6. Un composé de formule I de la revendication 1, dans laquelle
   $R_1$ et $R_2$ représentent l'hydrogène;
   $R_3$ représente un groupe alkyle en C 1 – C 3, un groupe méthyle substitué par le fluor, le chlore, le brome ou un groupe hydroxy, un groupe cyclopropyle ou un groupe cyclopropyle substitué par un groupe méthyle, le fluor, le chlore ou le brome;
   $R_4$ représente un groupe cycloalkyle en C 3 – C 4 ou un groupe cycloalkyle en C 3 – C 4 portant jusqu'à trois substituants identiques ou différents choisis parmi les groupes méthyle, le fluor, le chlore et le brome.

7. Un composé selon revendication 3, choisi parmi les suivants :
   2 – phénylamino – 4 – méthyl – 6 – cyclopropyl – pyrimidine,
   2 – phénylamino – 4 – éthyl – 6 – cyclopropyl – pyrimidine,
   2 – phénylamino – 4 – méthyl – 6 – (2 – méthylcyclopropyl) – pyrimidine et
   2 – (p – fluorophénylamino) – 4 – méthyl – 6 – cyclopropyl – pyrimidine.

8. Un composé selon revendication 1, choisi parmi les suivants :
   2 – phénylamino – 4,6 – bis – (cyclopropyl) – pyrimidine,
   2 – phénylamino – 4 – hydroxyméthyl – 6 – cyclopropyl – pyrimidine,
   2 – phénylamino – 4 – fluorométhyl – 6 – cyclopropyl – pyrimidine,
   2 – phénylamino – 4 – hydroxyméthyl – 6 – (2 – méthylcyclopropyl) – pyrimidine,
   2 – phénylamino – 4 – méthyl – 6 – (2 – fluorocyclopropyl) – pyrimidine,
   2 – phénylamino – 4 – méthyl – 6 – (2 – chlorocyclopropyl) – pyrimidine,
   2 – phénylamino – 4 – méthyl – 6 – (2 – difluorocyclopropyl) – pyrimidine,
   2 – phénylamino – 4 – fluorométhyl – 6 – (2 – fluorocyclopropyl) – pyrimidine,
   2 – phénylamino – 4 – fluorométhyl – 6 – (2 – chlorocyclopropyl) – pyrimidine,
   2 – phénylamino – 4 – fluorométhyl – 6 – (2 – méthylcyclopropyl) – pyrimidine et
   2 – phénylamino – 4 – éthyl – 6 – (2 – méthylcyclopropyl) – pyrimidine.

9. Procédé de préparation d'un composé de formule I, caractérisé en ce que :
   1) on fait réagir un sel de phénylguanidine de formule IIa

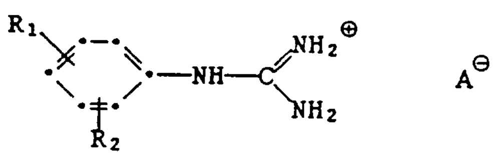

(IIa)

ou la guanidine dont il dérive, de formule IIb

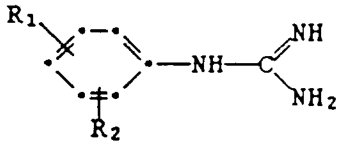

(IIb)

avec une dicétone de formule III

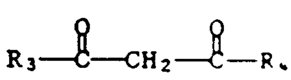

(III)

avec ou sans solvant, à des températures de 60 à 160 ° C,

2) on fait réagir l'urée avec une dicétone de formule III en présence d'un acide dans un solvant, à des températures de 20 à 140 ° C, puis on cyclise à la température de reflux en un dérivé de pyrimidine de formule V

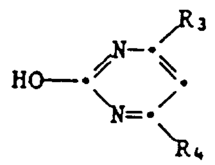

(V)

dont on échange le groupe OH contre un halogène à l'aide d'un exces de $POHal_3$ en présence ou en l'absence de solvant, à des températures de 50 à 110 ° C, ce qui donne un composé de formule

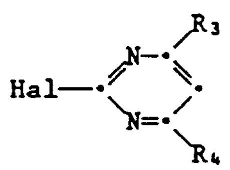

(VI)

dans laquelle Hal représente un halogène, puis on fait réagir le composé de formule VI avec un dérivé d'aniline de formule VII

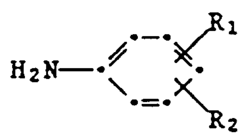

(VII)

à une température de 60 à 120 ° C, selon les conditions opératoires,

a) soit en présence d'un accepteur de protons avec ou sans solvant,

b) soit en présence d'un acide dans un solvant inerte; ou bien

3) on cyclise un sel de guanidine de formule VIII

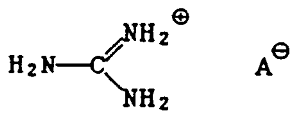

(VIII)

à l'aide d'une dicétone de formule III

a) sans solvant, à des températures de 100 à 160 ° C, ou bien

b) dans un solvant inerte, à des températures de 30 à 140 ° C, en un dérivé de pyrimidine de formule IX

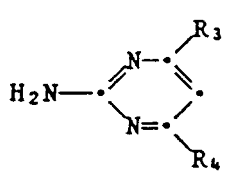

(IX)

qu'on fait réagir avec un composé de formule X

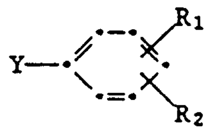

(X)

avec scission de HY, en présence d'un accepteur de protons, dans des solvants aprotoniques, à des températures de 30 à 140 ° C, les symboles $R_1$ à $R_4$ ayant dans les formules II à X les significations indiquées en référence à la formule I, $A^{\ominus}$ représentant un anion d'acide et Y un halogène.

**10.** Produit pour combattre ou prévenir une attaque par des insectes ou des microorganismes nuisibles, caractérisé en ce qu'il contient au moins un composant actif consistant en un composé de formule I de la revendication 1, avec une véhicule approprié.

**11.** Produit selon revendication 10, caractérisé en ce qu'il contient au moins un composant actif consistant en un composé de formule I selon revendication 2.

**12.** Produit selon revendication 10, caractérisé en ce qu'il contient au moins un composant actif consistant en un composé de formule I selon revendication 3.

**13.** Produit selon revendication 10, caractérisé en ce qu'il contient au moins un composant actif consistant en un composé de formule I selon revendication 4.

**14.** Produit selon revendication 10, caractérisé en ce qu'il contient au moins un composant actif consistant en un composé de formule I selon revendications 5 à 8.

**15.** Procédé pour combattre ou prévenir une attaque de végétaux cultivés par des insectes nuisibles ou des microorganismes phytopathogènes, caractérisé en ce que l'on applique sur les plantes, des parties des plantes ou leur habitat, en tant que substance active, un composé de formule I de la revendication 1.

**16.** Procédé selon la revendication 15, caractérisé en ce que l'on applique en tant que substance active un composé selon l'une des revendications 2 à 8.

**17.** Procédé selon la revendication 15, caractérisé en ce que l'on combat des mycètes phytopathogènes.

**18.** Procédé de préparation d'un produit agrochimique selon revendication 10, caractérisé en ce que l'on mélange intimement au moins un composé de formule I selon revendication 1 avec des additifs solides ou liquides appropriés et/ou des agents tensioactifs.

**19.** Un composé de formule XXI

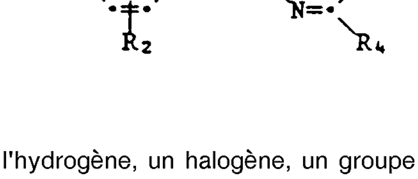

(XXI)

dans laquelle

- $R_1$ représente l'hydrogène, un halogène, un groupe alkyle en C 1 – C 3, trifluorométhyle, alcoxy en C 1 – C 3 ou halogénoalcoxy en C 1 – C 3;
- $R_2$ représente l'hydrogène, un halogène, un groupe alkyle en C 1 – C 3, trifluorométhyle ou alcoxy en C 1 – C 3;
- $R_4$ représente un groupe cycloalkyle en C 3 – C 6 ou un groupe cycloalkyle en C 3 – C 6 portant

jusqu'à trois substituants identiques ou différents choisis parmi les groupes méthyle et les halogènes.

**Revendications pour l'Etat contractant suivant : ES**

**1.** Procédé de préparation d'un composé de formule I

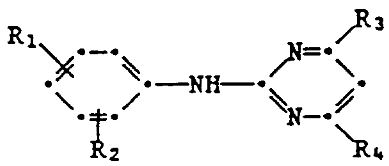

(I)

dans laquelle

$R_1$ représente l'hydrogène, un halogène, un groupe alkyle en C 1 – C 3, trifluorométhyle, alcoxy en C 1 – C 3 ou halogénoalcoxy en C 1 – C 3;

$R_2$ représente l'hydrogène, un halogène, un groupe alkyle en C 1 – C 3, trifluorométhyle ou alcoxy en C 1 – C 3;

$R_3$ représente l'hydrogène, un groupe alkyle en C 1 – C 4, ou un groupe alkyle en C 1 – C 4 substitué par un halogène ou un groupe hydroxy, un groupe cyclopropyle ou un groupe cyclopropyle portant jusqu'à trois substituants identiques ou différents choisis parmi les groupes méthyle et les halogènes;

$R_4$ représente un groupe cycloalkyle en C 3 – C 6 ou un groupe cycloalkyle en C 3 – C 6 portant jusqu'à trois substituants identiques ou différents choisis parmi les groupes méthyle et les halogènes,

caractérisé en ce que :

1) on fait réagir un sel de phénylguanidine de formule IIa

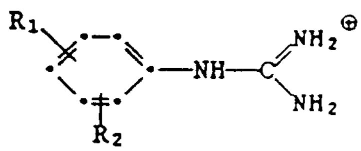

$A^{\ominus}$

(IIa)

ou la guanidine dont il dérive, de formule IIb

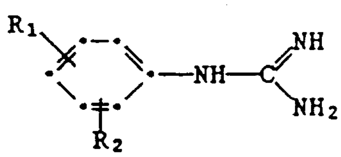

(IIb)

avec une dicétone de formule III

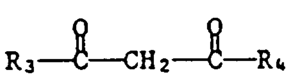

(III)

avec ou sans solvant, à des températures de 60 à 160 ° C,

2) on fait réagir l'urée avec une dicétone de formule III en présence d'un acide dans un solvant, à des températures de 20 à 140 ° C, puis on cyclise à la température de reflux en un dérivé de

pyrimidine de formule V

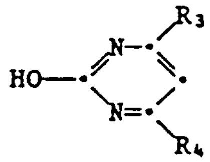

(V)

dont on échange le groupe OH contre un halogène à l'aide d'un excès de $POHal_3$ en présence ou en l'absence de solvant, à des températures de 50 à 110 ° C, ce qui donne un composé de formule

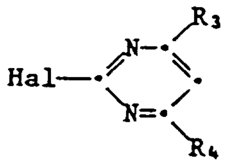

(VI)

dans laquelle Hal représente un halogène, puis on fait réagir le composé de formule VI avec un dérivé d'aniline de formule VII

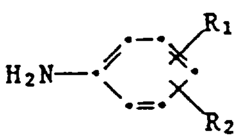

(VII)

à une température de 60 à 120 ° C, selon les conditions opératoires,

a) soit en présence d'un accepteur de protons avec ou sans solvant,

b) soit en présence d'un acide dans un solvant inerte; ou bien

3) on cyclise un sel de guanidine de formule VIII

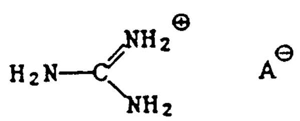

(VIII)

à l'aide d'une dicétone de formule III

a) sans solvant, à des températures de 100 à 160 ° C, ou bien

b) dans un solvant inerte, à des températures de 30 à 140 ° C, en un dérivé de pyrimidine de formule IX

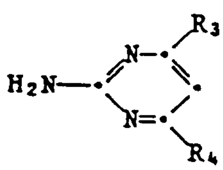

(IX)

qu'on fait réagir avec un composé de formule X

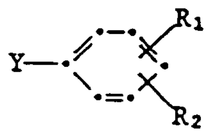

(X)

avec scission de HY, en présence d'un accepteur de protons, dans des solvants aprotoniques, à des températures de 30 à 140 °C, les symboles $R_1$ à $R_4$ ayant dans les formules II à X les significations indiquées en référence à la formule I, $A^{\ominus}$ représentant un anion d'acide et Y un halogène.

**2.** Procédé selon la revendication 1, caractérisé en ce que l'on prépare des composés dans lesquels $R_1$ et $R_2$ représentent l'hydrogène et les autres symboles ont les significations indiquées ci-dessus.

**3.** Procédé selon revendication 1, caractérisé en ce que l'on prépare des composés dans lesquels :
- $R_1$ représente l'hydrogène, un halogène, un groupe alkyle en C 1-C 3, trifluorométhyle, alcoxy en C 1-C 3 ou halogénoalcoxy en C 1-C 3,
- $R_2$ représente l'hydrogène, un halogène, un groupe alkyle en C 1-C 3, trifluorométhyle ou alcoxy en C 1-C 3,
- $R_3$ représente l'hydrogène, un groupe alkyle en C 1-C 4 ou un groupe alkyle en C 1-C 4 substitué par un halogène,
- $R_4$ représente un groupe cycloalkyle, en C 3-C 6 ou un groupe cycloalkyle en C 3-C 6 substitué par un groupe méthyle ou un halogène.

**4.** Procédé selon la revendication 1, caractérisé en ce que l'on prépare des composés dans lesquels :
- $R_1$ représente l'hydrogène, le fluor, le chlore, le brome, un groupe méthyle, éthyle, trifluorométhyle, méthoxy, éthoxy ou halogénométhoxy;
- $R_2$ représente l'hydrogène, le fluor, le chlore, un groupe méthyle, trifluorométhyle, méthoxy ou éthoxy;
- $R_3$ représente l'hydrogène, un groupe méthyle, éthyle, n-propyle ou sec-butyle; ou un groupe méthyle substitué par le fluor, le chlore ou le brome;
- $R_4$ représente un groupe cycloalkyle en C 3-C 6 ou un groupe cycloalkyle en C 3-C 6 substitué par un groupe méthyle, le fluor, le chlore ou le brome.

**5.** Procédé selon revendication 1, caractérisé en ce que l'on prépare des composés dans lesquels :
- $R_1$ représente l'hydrogène, le fluor, le chlore, un groupe méthyle, trifluorométhyle, méthoxy ou difluorométhoxy;
- $R_2$ représente l'hydrogène, le fluor, le chlore, un groupe méthyle, trifluorométhyle ou méthoxy;
- $R_3$ représente l'hydrogène, un groupe alkyle en C 1-C 3, un groupe alkyle en C 1-C 2 substitué par un halogène ou un groupe hydroxy, un groupe cyclopropyle ou cyclopropyle substitué par un groupe méthyle ou un halogène;
- $R_4$ représente un groupe cycloalkyle en C 3-C 6 ou un groupe cycloalkyle en C 3-C 4 portant jusqu'à trois substituants identiques ou différents choisis parmi les groupes méthyle et les halogènes.

**6.** Procédé selon revendication 1, caractérisé en ce que l'on prépare des composés dans lesquels :
- $R_1$ et $R_2$ représentent l'hydrogène;
- $R_3$ représente un groupe alkyle en C 1-C 3, un groupe méthyle substitué par le fluor, le chlore, le brome ou un groupe hydroxy, un groupe cyclopropyle ou un groupe cyclopropyle substitué par un groupe méthyle, le fluor, le chlore ou le brome;
- $R_4$ représente un groupe cycloalkyle en C 3-C 4 ou un groupe cycloalkyle en C 3-C 4 portant jusqu'à trois substituants identiques ou différents choisis parmi les groupes méthyle, le fluor, le chlore et le brome.

**7.** Procédé selon revendication 1, caractérisé en ce que l'on prépare un composé choisi parmi les suivants :
2-phénylamino-4-méthyl-6-cyclopropyl-pyrimidine,

2 – phénylamino – 4 – éthyl – 6 – cyclopropyl – pyrimidine,
2 – phénylamino – 4 – méthyl – 6 – (2 – méthylcyclopropyl) – pyrimidine et
2 – (p – fluorophénylamino) – 4 – méthyl – 6 – cyclopropyl – pyrimidine.

**8.** Procédé selon revendication 1, caractérisé en ce que l'on prépare un composé choisi parmi les suivants :
2 – phénylamino – 4,6 – bis – (cyclopropyl) – pyrimidine,
2 – phénylamino – 4 – hydroxyméthyl – 6 – cyclopropyl – pyrimidine,
2 – phénylamino – 4 – fluorométhyl – 6 – cyclopropyl – pyrimidine,
2 – phénylamino – 4 – hydroxyméthyl – 6 – (2 – méthylcyclopropyl) – pyrimidine,
2 – phénylamino – 4 – méthyl – 6 – (2 – fluorocyclopropyl) – pyrimidine,
2 – phénylamino – 4 – méthyl – 6 – (2 – chlorocyclopropyl) – pyrimidine,
2 – phénylamino – 4 – méthyl – 6 – (2 – difluorocyclopropyl) – pyrimidine,
2 – phénylamino – 4 – fluorométhyl – 6 – (2 – fluorocyclopropyl) – pyrimidine,
2 – phénylamino – 4 – fluorométhyl – 6 – (2 – chlorocyclopropyl) – pyrimidine,
2 – phénylamino – 4 – fluorométhyl – 6 – (2 – méthylcyclopropyl) – pyrimidine et
2 – phénylamino – 4 – éthyl – 6 – (2 – méthylcyclopropyl) – pyrimidine.

**9.** Produit pour combattre ou prévenir une infestation par des insectes ou des microorganismes nuisibles, caractérisé en ce qu'il contient, avec un véhicule approprié, au moins un composant actif consistant en un composé de formule I

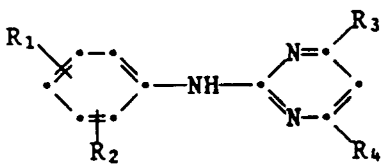

(I)

dans laquelle :

$R_1$ représente l'hydrogène, un halogène, un groupe alkyle en C 1 – C 3, trifluorométhyle, alcoxy en C 1 – C 3 ou halogénoalcoxy en C 1 – C 3;
$R_2$ représente l'hydrogène, un halogène, un groupe alkyle en C 1 – C 3, trifluorométhyle ou alcoxy en C 1 – C 3;
$R_3$ représente l'hydrogène, un groupe alkyle en C 1 – C 4, ou un groupe alkyle en C 1 – C 4 substitué par un halogène ou un groupe hydroxy, un groupe cyclopropyle ou un groupe cyclopropyle portant jusqu'à trois substituants identiques ou différents choisis parmi les groupes méthyle et les halogènes;
$R_4$ représente un groupe cycloalkyle en C 3 – C 6 ou un groupe cycloalkyle en C 3 – C 6 portant jusqu'à trois substituants identiques ou différents choisis parmi les groupes méthyle et les halogènes.

**10.** Produit selon revendication 9, caractérisé en ce qu'il contient au moins un conposant actif consistant en un composé de formule I dans laquelle $R_3$ et $R_4$ ont les significations indiquées ci – dessus et $R_1$ et $R_2$ représentent l'hydrogène.

**11.** Produit selon revendication 9, caractérisé en ce qu'il contient au moins un composant actif consistant en un composé de formule I dans laquelle

$R_1$ représente l'hydrogène, un halogène, un groupe alkyle en C 1 – C 3, trifluorométhyle, alcoxy en C 1 – C 3 ou halogénoalcoxy en C 1 – C 3,
$R_2$ représente l'hydrogène, un halogène, un groupe alkyle en C 1 – C 3, trifluorométhyle ou alcoxy en C 1 – C 3,
$R_3$ représente l'hydrogène, un groupe alkyle en C 1 – C 4 ou un groupe alkyle en C 1 – C 4 substitué par un halogène,
$R_4$ représente un groupe cycloalkyle en C 3 – C 6 ou un groupe cycloalkyle en C 3 – C 6 substitué par un groupe méthyle ou un halogène.

**12.** Produit selon revendication 9, caractérisé en ce qu'il contient au moins un composant actif consistant en un composé de formule I dans laquelle

$R_1$ représente l'hydrogène, le fluor, le chlore, le brome, un groupe méthyle, éthyle, trifluorométhyle, méthoxy, éthoxy ou halogénométhoxy;

$R_2$ représente l'hydrogène, le fluor, le chlore, un groupe méthyle, trifluorométhyle, méthoxy ou éthoxy;

$R_3$ représente l'hydrogène, un groupe méthyle, éthyle, n-propyle ou sec-butyle; ou un groupe méthyle substitué par le fluor, le chlore ou le brome;

$R_4$ représente un groupe cycloalkyle en C 3-C 6 ou un groupe cycloalkyle en C 3-C 6 substitué par un groupe méthyle, le fluor, le chlore ou le brome.

**13.** Produit selon revendication 9, caractérisé en ce qu'il contient au moins un composant actif consistant en un composé de formule I dans laquelle :

$R_1$ représente l'hydrogène, le fluor, le chlore, un groupe méthyle, trifluorométhyle, méthoxy ou difluorométhoxy;

$R_2$ représente l'hydrogène, le fluor, le chlore, un groupe méthyle, trifluorométhyle ou méthoxy;

$R_3$ représente l'hydrogène, un groupe alkyle en C 1-C 3, un groupe alkyle en C 1-C 2 substitué par un halogène ou un groupe hydroxy, un groupe cyclopropyle ou cyclopropyle substitué par un groupe méthyle ou un halogène;

$R_4$ représente un groupe cycloalkyle en C 3-C 6 ou un groupe cycloalkyle en C 3-C 4 portant jusqu'à trois substituants identiques ou différents choisis parmi les groupes méthyle et les halogènes.

**14.** Produit selon revendication 9, caractérisé en ce qu'il contient au moins un composant actif consistant en un composé de formule I dans laquelle :

$R_1$ et $R_2$ représentent l'hydrogène;

$R_3$ représente un groupe alkyle en C 1-C 3, un groupe méthyle substitué par le fluor, le chlore, le brome ou un groupe hydroxy, un groupe cyclopropyle ou un groupe cyclopropyle substitué par un groupe méthyle, le fluor, le chlore ou le brome;

$R_4$ représente un groupe cycloalkyle en C 3-C 4 ou un groupe cycloalkyle en C 3-C 4 portant jusqu'à trois substituants identiques ou différents choisis parmi les groupes méthyle, le fluor, le chlore et le brome.

**15.** Produit selon revendication 9, caractérisé en ce qu'il contient au moins un composant actif consistant en un composé de formule I choisi parmi les suivants :

2-phénylamino-4-méthyl-6-cyclopropyl-pyrimidine,
2-phénylamino-4-éthyl-6-cyclopropyl-pyrimidine,
2-phénylamino-4-méthyl-6-(2-méthylcyclopropyl)-pyrimidine et
2-(p-fluorophénylamino)-4-méthyl-6-cyclopropyl-pyrimidine.

**16.** Produit selon revendication 9, caractérisé en ce qu'il contient au moins un composant actif consistant en un composé de formule I choisi parmi les suivants :

2-phénylamino-4,6-bis-(cyclopropyl)-pyrimidine,
2-phénylamino-4-hydroxyméthyl-6-cyclopropyl-pyrimidine,
2-phénylamino-4-fluorométhyl-6-cyclopropyl-pyrimidine,
2-phénylamino-4-hydroxyméthyl-6-(2-méthylcyclopropyl)-pyrimidine,
2-phénylamino-4-méthyl-6-(2-fluorocyclopropyl)-pyrimidine,
2-phénylamino-4-méthyl-6-(2-chlorocyclopropyl)-pyrimidine,
2-phénylamino-4-méthyl-6-(2-difluorocyclopropyl)-pyrimidine,
2-phénylamino-4-fluorométhyl-6-(2-fluorocyclopropyl)-pyrimidine,
2-phénylamino-4-fluorométhyl-6-(2-chlorocyclopropyl)-pyrimidine,
2-phénylamino-4-fluorométhyl-6-(2-méthylcyclopropyl)-pyrimidine et
2-phénylamino-4-éthyl-6-(2-méthylcyclopropyl)-pyrimidine.

**17.** Procédé pour combattre ou prévenir une infestation de végétaux cultivés par des insectes nuisibles ou des microorganismes phytopathogènes, caractérisé en ce que l'on applique sur les végétaux, des parties des végétaux ou leur habitat, en tant que substance active, un composé de formule I selon revendication 9.

**18.** Procédé selon revendication 17, caractérisé en ce que l'on applique en tant que substance active un composé selon l'une des revendications 10 à 16.

**19.** Procédé selon revendication 17, caractérisé en ce que l'on combat des mycètes phytopathogènes.

**20.** Procédé de préparation d'un produit agrochimique selon revendication 9, caractérisé en ce que l'on mélange intimement au moins un composé de formule I selon revendication 9 avec des additifs solides ou liquides appropriés et/ou des agents tensioactifs.